# FASCICULE DE BREVET EUROPEEN

(11) **EP 2 010 562 B1**
(45) Date de publication et mention de la délivrance du brevet: **08.02.2012**
(21) Numéro de dépôt: 07731380.7
(22) Date de dépôt: 27.04.2007
(51) Int. Cl.: C07K 14/45, C07K 19/00, C12N 15/30, A61K 38/16, A61K 39/002, A61P 17/00, A61P 37/00

(54) **NOUVEAUX POLYPEPTIDES INDUISANT LA MIGRATION DES CELLULES DENDRITIQUES, AINSI QUE LES MEDICAMENTS ET COMPOSITIONS PHARMACEUTIQUES CONTENANT DE TELS POLYPEPTIDES**
NEUE, DIE MIGRATION DENDRITISCHER ZELLEN INDUZIERENDE POLYPEPTIDE, SOWIE MEDIKAMENTE UND PHARMAZEUTISCHE ZUSAMMENSETZUNGEN, DIE DIESE ENTHALTEN
NOVEL POLYPEPTIDES INDUCING DENDRITIC CELL MIGRATION, AS WELL AS MEDICINES AND PHARMACEUTICAL COMPOSITIONS CONTAINING SAME

(30) Priorité: 27.04.2006 FR 0603824
(43) Date de publication de la demande: 07.01.2009
(73) Titulaire: Centre National de la Recherche Scientifique, 75016 Paris (FR); INSTITUT NATIONAL DE LA SANTE ET DE LA RECHERCHE MEDICALE (INSERM), 75013 Paris (FR); UNIVERSITE CLAUDE BERNARD - LYON 1, 69622 Villeurbanne Cédex (FR)
(72) Inventeur: PERSAT, Florence, F-69500 Bron (FR); VINCENT, Claude, F-69001 Lyon (FR); CESBRON-DELAUW, Marie-France, F-38700 La Tronche (FR)
(74) Mandataire: Sarlin, Laure V.
(86) Numéro de dépôt international: PCT/FR2007/000728
(87) Numéro de publication internationale: WO 2007/125209

(56) Documents cités:
- EP-A- 0 353 111
- DIANA J ET AL: "Migration and maturation of human dendritic cells infected with Toxoplasma gondii depend on parasite strain type" FEMS IMMUNOLOGY AND MEDICAL MICROBIOLOGY, ELSEVIER SCIENCE B.V., AMSTERDAM, NL, vol. 42, no. 3, 1 novembre 2004 (2004-11-01), pages 321-331, XP004597795 ISSN: 0928-8244
- DIANA J. ET AL.: "Toxoplasma gondii regulates recuitement amd migration of human dendritic cells via different soluble secreted factors" CLINICAL AND EXPERIMENTAL IMMUNOLOGY, vol. 141, 2005, pages 475-484, XP002399478

## Description

La présente invention concerne le domaine technique du traitement des maladies allergiques et auto-immunes. En particulier, la présente invention a pour objet de nouveaux polypeptides qui induisent la migration des cellules dendritiques, les acides nucléiques codant pour de tels polypeptides, ainsi que les médicaments et compositions pharmaceutiques contenant de tels polypeptides.

*Toxoplasma gondii* est un parasite protozoaire à l'origine de la toxoplasmose. Il a été démontré que dans certaines conditions les parasites de *Toxoplasma gondii,* quelque soit la souche utilisée, sécrétaient des antigènes d'excrétion-sécrétion dont certains, nommés GRA, proviennent des granules denses. Les gènes GRA1, GRA2, GRAS, et GRA6, qui codent pour des antigènes d'excrétion-sécrétion de *Toxoplasma gondii,* sont décrits dans les publications suivantes :
- GRA1 (P23) : CESBRON-DELAUW et al. (Proc. Natl. Acad. Sci. 1989, 86, p. 7537-7541) WO89/05658
- GRA2 (GP28.5) : FR 2692282, FR 2702491 ; MERCIER et al., Mol. Biochem. Parasitol., 1993, 58, 71-82)
- GRA5 (P21) : LECORDIER et al. (Mol. Biochem. Parasitol., 1993, 59, 143-154); FR 2702491. La protéine GRAS (ou P21, 21 kDa) est caractérisée par un peptide signal, deux régions hydrophiles N- et C-terminales flanquant un domaine central hydrophobe (Lecordier et al., Mol. Biochem. Parasitol. 1993, 59, 143-154). La séquence en acides aminés de la protéine GRAS (120 aa) ne présente pas de similitude avec des protéines de fonction connue. Pour déterminer la fonction de GRAS, un mutant de toxoplasme invalidé pour le gène GRA5 (nommé KO GRAS) a été isolé. Les parasites KO GRAS présentent une croissance intracellulaire identique à la souche RH sauvage. Leur virulence chez la souris n'apparaît pas modifiée (Mercier et al., Mol. Biochem. Parasitol. 2001, 116, 247-251). La protéine GRAS sécrétée par les tachyzoites de la souche R_{H} a la séquence **SEQ ID N°1 :** 
MASVKRVVVAVMIVNVLALIFVGVAGSTRDVGSGGDDSEGARGRE
QQQVQQHEQNEDRSLFERGRAAVTGHPVRTAVGLAAAVVAVVSLL
RLLKRRRRRAIQEESKESATAEEEEVAEEE
(déposée dans la banque UniProtKB/Swiss-Prot sous le numéro AC Q07828) et, dans la suite, nommée GRA5-RH.
La séquence ADN **SEQ ID N°2 :** 
ATGGCGTCTGTAAAACGCGTCGTTGTGGCGGTAATGATCGTGAACGTGCTGGCTTTAATTTT
TGTGGGCGTTGCCGGTTCAACGCGTGACGTAGGGTCAGGCGGGGATGACTCCGAAGGTGCTA
GGGGGCGTGAACAACAACAGGTACAACAACACGAACAAAATGAAGACCGATCGTTATTCGAA
AGGGGAAGAGCAGCGGTGACTGGACATCCAGTGAGGACTGCAGTGGGACTTGCTGCAGCTGT
GGTGGCCGTTGTGTCACTACTGCGATTGTTGAAAAGGAGGAGAAGACGCGCGATTCAAGAAG
AGAGCAAGGAGTCTGCAACCGCGGAAGAGGAAGAAGTTGCCGAGGAAGAGTAA
 codant pour GRA5-RH est répertoriée dans GenBank avec le numéro d'accession : L06091. - GRA6 (P32) : FR 2702491.

Les protéines GRA constituent une famille de molécules de petit poids moléculaire (entre 20 et 40 kDa). Ces protéines sont stockées dans les granules denses des toxoplasmes et sont sécrétées dans la vacuole parasitophore. Jusqu'à présent 9 protéines de granules denses de la forme tachyzoïte ont été caractérisées (GRA1 à GRA9) (Mercier et al., Int J. Parasitol., 2005, 35, 829-849). Le stimulus biologique qui déclenche l'exocytose des protéines GRA dans le compartiment vacuolaire, à la suite de l'installation des parasites dans la cellule-hôte, n'est pas connu à l'heure actuelle. Par contre, une décharge massive du contenu des granules denses peut être induite en condition acellulaire, après incubation des parasites dans un milieu complémenté en sérum (ESA) (Darcy et al., Parasite Immunol. 1988 , 10, 553-567). Dans ces conditions, la quantité de protéines GRA sécrétées est dépendante de la concentration en protéines sériques (10 à 40 µg / mg de protéines totales / heure pour 10 à 40% de sérum) (Coppens et al., Eur. J. Cell Biol. 1999, 78, 463-472). Dans ces conditions, les protéines GRA se retrouvent dans le milieu sous forme essentiellement soluble (Lecordier et al., Mol. Biol Cell 1999,10, 1277-1287).

Différentes études ont été menées par les inventeurs (Diana et al., Clinical and Experimental immunology, 2005, 1-9 et FEMS Immunology and Medical Microbiology, 2004, 42, 321-331) sur le surnageant sécrété par différentes souches de *Toxoplasma gondii* et ont démontré que ce dernier avait une influence sur la migration et la maturation des cellules dendritiques *in vitro.*

Dans le cadre de l'invention, les inventeurs ont mis en évidence qu'un polypeptide correspondant à la partie *N*-terminale de la protéine GRA5 portait l'activité d'induction de migration et serait susceptible d'induire la déplétion d'un tissu en cellules dendritiques.

En particulier, la présente invention a pour objet le polypeptide correspondant aux acides aminés 26 à 75 de la séquence de la protéine GRA5-RH de **SEQ ID N°1** : 
MASVKRVVVAVMIVNVLALIFVGVAGSTRDVGSGGDDSEGARGRE
QQQVQQHEQNEDRSLFERGRAAVTGHPVRTAVGLAAAVVAVVSLL
RLLKRRRRRAIQEESKESATAEEEEVAEEE
 ses variants, homologues, ainsi que leurs fragments, actifs sur la migration des cellules dendritiques.

Les acides aminés 26 à 75 de la séquence de la protéine GRA5-RH correspondent à la séquence **SEQ ID N°3** :
GSTRDVGSGG DDSEGARGRE QQQVQQHEQN EDRSLFERGR AAVTGHPVRTL'invention vise, en plus du polypeptide de SEQ ID N°3, les variants, homologues et fragments de ce polypeptide, ainsi que les fragments desdits variants et homologues, lesdits fragments, homologues et variants étant actifs sur la migration des cellules dendritiques.

En particulier, les polypeptides selon l'invention sont choisis parmi les polypeptides de séquence : 
SEQ ID N° 3: GSTRDVGSGG DDSEGARGRE QQQVQQHEQN EDRSLFERGR
AAVTGHPVRT
 
SEQ ID N° 4: GSTRDTGSGG DDSEGAWGGE QQQVQQHGQS EDRSLFERGR
AAVTGHPVRT
 
SEQ ID N° 5: GSTCDTGSGG DDSEGAWGGE QQQVQQHGQS EDRSLFERGR
AAVTGHPVRT
 **SEQ ID N° 6:** GSTRDVGSGA DDSEGAGGRE RQQVQQHEQN EDRSLFERGR AAVTGHPVRT leurs homologues ou fragments, actifs sur la migration des cellules dendritiques.

De façon préférée, les polypeptides selon l'invention sont choisis parmi les polypeptides de séquence **SEQ ID N°3, SEQ ID N°4, SEQ ID N°5 et SEQ ID N°6** et les polypeptides de séquence **SEQ ID N°3, SEQ ID N°4, SEQ ID N°5 et SEQ ID N°6** dans lesquelles l'acide aminé (la thréonine) C-terminal a été supprimé.

Dans le cadre de la description de l'invention, les termes ci-dessous ont les significations suivantes.

Les termes « polypeptide » ou « protéine » sont utilisés indifféremment dans le cadre de l'invention, pour désigner un enchaînement d'acides aminés, sans qu'aucune limitation inférieure ou supérieure de taille soit associée à l'un de ces termes.

Par « protéine GRAS-RH », on désigne la protéine GRA de poids moléculaire d'environ 21 KDa sécrétée par des tachyzoites de *Toxoplasma gondii* de la souche RH. Cette sécrétion peut notamment avoir lieu dans les conditions décrites par Darcy *et al. (supra).* La protéine GRA5 sécrétée par les tachyzoites de la souche RH, a la séquence **SEQ ID N°1 :** 
MASVKRVVVAVMIVNVLALIFVGVAGSTRDVGSGGDDSEGARGRE
QQQVQQHEQNEDRSLFERGRAAVTGHPVRTAVGLAAAVVAVVSLL
RLLKRRRRRAIQEESKESATAEEEEVAEEE
 et nommée GRA5-RH. La séquence ADN **SEQ ID N°2** : 
ATGGCGTCTGTAAAACGCGTCGTTGTGGCGGTAATGATCGTGAACGTGCTGGCTTTAATTTT
TGTGGGCGTTGCCGGTTCAACGCGTGACGTAGGGTCAGGCGGGATGACTCCGAAGGTGCTA
GGGGGCGTGAACAACAACAGGTACAACAACACGAACAAAATGAAGACCGATCGTTATTCGAA
AGGGGAAGAGCAGCGGTGACTGGACATCCAGTGAGGACTGCAGTGGGACTTGCTGCAGCTGT
GGTGGCCGTTGTGTCACTACTGCGATTGTTGAAAAGGAGGAGAAGACGCGCGATTCAAGAAG
AGAGCAAGGAGTCTGCAACCGCGGAAGAGGAAGAAGTTGCCGAGGAAGAGTAA
 codant pour GRAS-RH est répertoriée dans GenBank avec le numéro d'accession : L06091.

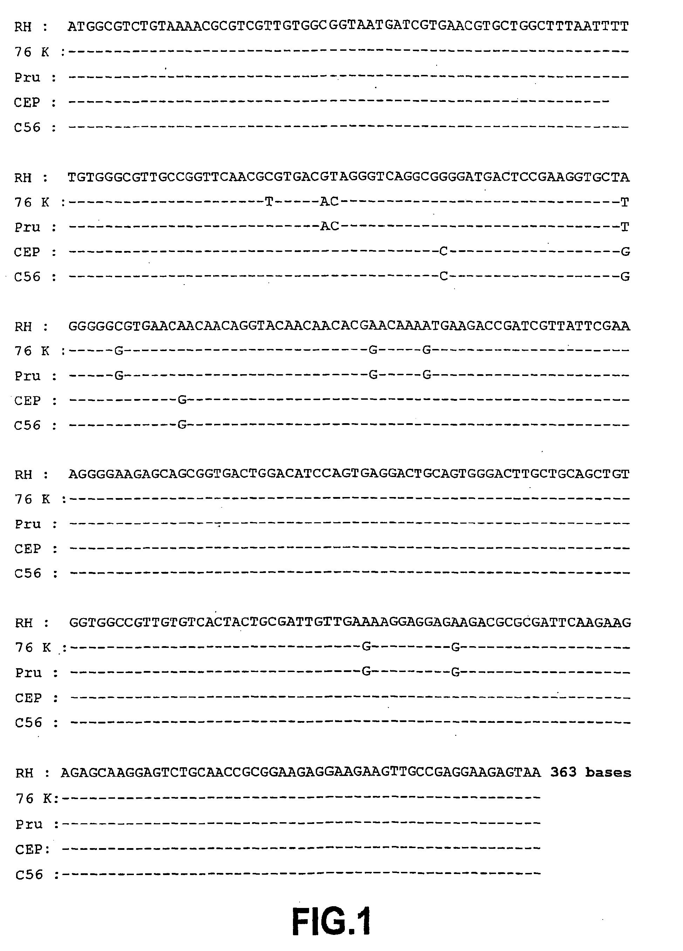

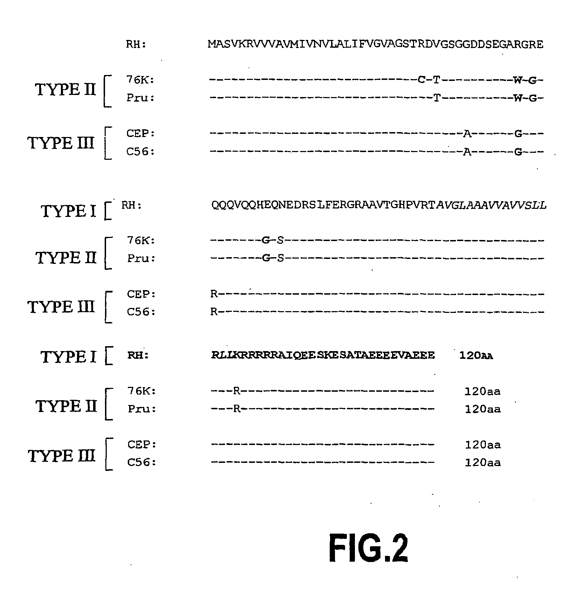

Par « variants de la protéine GRA5-RH », on désigne tous les variants alléliques de la protéine GRAS-RH, c'est-à-dire toutes les protéines GRAS sécrétées, par des tachyzoites de *Toxoplasma gondii,* autre que ceux de la souche RH, par exemple dans les conditions décrites par Darcy *et al. (supra)* ou par les inventeurs (Diana *et al.,* 2005, *supra).* Ces protéines GRAS diffèrent, le plus souvent, de la protéine GRA5 sécrétées par la souche RH par au moins une délétion, addition ou substitution d'au moins un acide aminé, une troncature, un allongement et/ou une fusion chimérique. On peut également considérer que ces variants se lient à l'anticorps monoclonal TG 17-113 (Charif, H., et al. Exp. Parasitol. 1993, 71, 114-124). Les tachyzoites issus de différentes souches peuvent sécréter de telles protéines GRA5. Les parasites *Toxoplasma gondii,* issus de différents génotypes (I, II ou III) sécrètent cette protéine. Les **Figures 1** et **2** montrent le gène de la protéine GRAS, chez différentes souches de *Toxoplasma gondii* (RH déposée à l'ATCC sous le n°50174, 76K décrite par Laugier M., et al. Ann. Parasitol. Hum. Comp. 1970, 45, 389-403, Pru décrite par Martrou P. et al. Limousin Médical. 1965, 53, 3-7, CEP déposée à l'ATCC sous le n°50842 ou C56 déposée à l'ATCC sous le n° 50951).

Les variants du polypeptide correspondant aux acides aminés 26 à 75 de la séquence d'une protéine GRA5-RH correspondent à un fragment d'une protéine GRAS, sécrétée par des tachyzoites issus d'une souche de *Toxoplasma gondii,* autre que la souche RH la sécrétion étant, par exemple, réalisée dans les conditions décrites par Darcy *et al. (supra).* Pour les souches autres que la souche RH, les positions 26 à 75 sont déterminées, après alignement avec la séquence GRAS-RH de **SEQ ID N°1,** et correspondent aux acides aminés de la séquence situés de la position 26 à la position 75 de la **SEQ ID N°1,** comme illustré **Figure 2**. Par conséquent, les polypeptides correspondant aux acides aminés 26 à 75 de la séquence d'une protéine GRAS ont les séquences suivantes pour les souches suivantes :
*SOUCHE RH* 
SEQ ID N° 3: GSTRDVGSGG DDSEGARGRE QQQVQQHEQN EDRSLFERGR
AAVTGHPVRT
*SOUCHE PRU* 
SEQ ID N° 4: GSTRDTGSGG DDSEGAWGGE QQQVQQHGQS EDRSLFERGR
AAVTGHPVRT
*SOUCHE 76K* 
SEQ ID N° 5: GSTCDTGSGG DDSEGAWGGE QQQVQQHGQS EDRSLFERGR
AAVTGHPVRT
*SOUCHE C56 ET SOUCHE CEP*

SEQ ID N° 6: GSTRDVGSGA DDSEGAGGRE RQQVQQHEQN EDRSLFERGR
AAVTGHPVRT

Les polypeptides de **SEQ ID N° 4**, **SEQ ID N° 5, SEQ ID N° 6** sont des exemples de variants du polypeptide de **SEQ ID N°3**.

Par polypeptides « homologues », on entend des polypeptides qui présentent notamment, par rapport au polypeptide dont ils sont l'homologue, au moins une délétion, addition ou substitution d'au moins un acide aminé, une troncature, un allongement et/ou une fusion chimérique.

Parmi les polypeptides homologues et variants, on préfère ceux dont la séquence d'acides aminés présente au moins 80% d'homologie, de préférence au moins 85%, au moins 87%, au moins 90%, au moins 93%, au moins 95%, au moins 97%, au moins 98%, au moins 99% et préférentiellement 100% d'homologie avec les séquences d'acides aminés des polypeptides dont ils sont l'homologue ou le variant. De préférence, les polypeptides homologues ou variants ne diffèrent du polypeptide de référence que par une, deux ou trois modifications d'acides aminés choisis parmi les additions, délétions ou substitutions. Dans le cas d'une substitution, un ou plusieurs acides aminés consécutifs ou non consécutifs, peuvent être remplacés par des acides aminés équivalents. L'expression acide aminé équivalent s'entend de tout acide aminé susceptible d'être substitué à l'un des acides aminés de la structure de base sans cependant modifier les caractéristiques ou propriétés fonctionnelles essentielles, comme leur activité sur la migration des cellules dendritiques. Les groupes d'amino acides ci-après sont généralement reconnus comme équivalents et correspondent à des substitutions conservatives qui conduisent à une forme de la protéine et à une fonction équivalente :
- Ala, Ser, Thr, Pro, Gly
- Asn, Asp, Glu, Gln,
- His, Arg, Lys,
- Met, Leu, Ile, Val,
- Phe, Tyr, Trp.

L'invention a également pour objet les fragments, actifs sur la migration des cellules dendritiques, du polypeptide de **SEQ ID N°3,** ainsi que les fragments actifs d'un homologue ou variant du polypeptide de **SEQ ID N°3.** De tels fragments comportent au minimum 15 acides aminés consécutifs, de préférence 17, 20, 23, 25 ou 30 amino-acides consécutifs. Des exemples de tels fragments sont les polypeptides de séquence **SEQ ID N°3, SEQ ID N°4, SEQ ID N°5 et SEQ ID N°6** dans lesquelles l'acide aminé (la thréonine) C-terminal a été supprimé.

Les fragments de polypeptide selon l'invention obtenus par clivage dudit polypeptide par une enzyme protéolytique, par un réactif chimique, ou encore en plaçant ledit polypeptide dans un environnement très acide font également partie de l'invention.

A titre d'autres exemples de fragments, on peut citer les peptides correspondant aux acides aminés 30 à 58, ou 37 à 63 de la séquence d'une protéine GRAS, et en particulier de la séquence d'une protéine GRAS des souches RH, PRU, 76K, CR6 ou CEP.

Dans le cas de la souche PRU, le fragment correspondant aux acides aminés 30 à 58 de la protéine GRA5-PRU présente la séquence :
**SEQ ID N°16** : DTGSGGDDSEGAWGGEQQQVQQHGQSEDR
le fragment correspondant aux acides aminés 37 à 63 de la protéine GRA5-PRU présente la séquence :
**SEQ ID N°17** : DSEGAWGGEQQQVQQHGQSEDRSLFERPar « protéine de fusion » de deux polypeptides, on entend un enchaînement colinéaire de deux polypeptides qui peuvent être liés éventuellement par un bras de liaison comportant, de préférence, de 2 à 6 acides aminés.

La présente invention a également pour objet les protéines de fusion entre la GST et un polypeptide tel que défini précédemment, ainsi que les homologues ou fragments de telles protéines, actifs sur la migration des cellules dendritiques.

De telles protéines de fusion sont, par exemple, la protéine de séquence **SEQ ID N°13** : 
S P I L G Y W K I K G L V Q P T R L L L E Y L E E K Y E E H L Y E R D E G D K W R N K
K F E L G L E F P N L P Y Y I D G D V K L T Q S M A I I R Y I A D K H N M L G G C P K
E R A E I S M L E G A V L D I R Y G V S R I A Y S K D F E T L K V D F L S K L P E M L K
M F E D R L C H K T Y L N G D H V T H P D F M L Y D A L D V V L Y M D P M C L D A F
P K L V C F K K R I E A I P Q I D K Y L K S S K Y I A W P L Q G W Q A T F G G G D H P
P K S D L I E G R G I P G G S T R D V G S G G D D S E G A R G R E Q Q V Q Q H E Q
N E D R S L F E R G R A A V T G H P V R E F I V T D
 la protéine de séquence **SEQ ID N°14 :** 
S P I L G Y W K I K G L V Q P T R L L L E Y L E E K Y E E H L Y E R D E G D K W R N K
K F E L G L E F P N L P Y Y I D G D V K L T Q S M A I I R Y I A D K H N M L G G C P K
E R A E I S M L E G A V L D I R Y G V S R I A Y S K D F E T L K V D F L S K L P E M L K
M F E D R L C H K T Y L N G D H V T H P D F M L Y D A L D V V L Y M D P M C L D A F
P K L V C F K K R I E A I P Q I D K Y L K S S K Y I A W P L Q G W Q A T F G G G D H P
 
PKSDLIEGRGIPGGSTCDTGSGGDDSEGAWGGEQQQVQQHGQ
SEDRSLFERGRAAVTGHPVREFIVTD
 la protéine de séquence **SEQ ID N°15** :

S P I L G Y W K I K G L V Q P T R L L L E Y L E E K Y E E H L Y E R D E G D K W R N K
K F E L G L E F P N L P Y Y I D G D V K L T Q S M A I I R Y I A D K H N M L G G C P K
E R A E I S M L E G A V L D I R Y G V S R I A Y S K D F E T L K V D F L S K L P E M L K
M F E D R L C H K T Y L N G D H V T H P D F M L Y D A L D V V L Y M D P M C L D A F
P K L V C F K K R I E A I P Q I D K Y L K S S K Y I A W P L Q G W Q A T F G G G D H P
P K S D L I E G R G I P G G S T R D V G S G A D D S E G A G G R E R Q Q V Q Q H E Q
N E D R S L F E R G R A A V T G H P V R E F I V T D

La séquence correspondant à la GST peut être clivée par incubation des protéines ci-dessus avec une protéase : le facteur Xa.

Par « actif sur la migration des cellules dendritiques », on entend un polypeptide qui induit la migration des cellules dendritiques, déterminée d'après le test *in vitro* détaillé à l'exemple ci-après, correspondant au moins à 70%, de préférence à au moins 80%, à au moins 90%, et préférentiellement à au moins 95% de l'activité du polypeptide de **SEQ ID N°3**. Le test de migration correspond à une première incubation des cellules dendritiques avec le polypeptide ou la fraction à tester, 24 h plus tard les cellules sont lavées et déposées à la partie supérieure d'une chambre de migration dont la partie inférieure contient la chimiokine MIP3-β. Les cellules qui sont passées du compartiment supérieur vers le compartiment inférieur sont comptées et le pourcentage qu'elles représentent par rapport au nombre de cellules déposées permet de quantifier la migration. L'activité d'un polypeptide correspond au pourcentage de cellules ayant migré. Dans le but de comparer des expériences différentes et de pouvoir effectuer un traitement statistique des données, les pourcentages de migration sont normalisés par le calcul d'un l'index de migration en utilisant la migration spontanée des cellules dendritiques comme référence (index de migration).

De préférence un polypeptide selon l'invention est un polypeptide constitué de la séquence **SEQ ID N° 3, SEQ ID N° 4**, **SEQ ID N° 5, SEQ ID N° 6, SEQ ID N° 16** ou **SEQ ID N° 17**, ou d'une séquence possédant au moins 80% d'homologie, de préférence au moins 85%, au moins 90%, au moins 95%, au moins 98% et préférentiellement au moins 99% d'homologie avec la **SEQ ID N° 3, SEQ ID N° 4**, **SEQ ID N° 5, SEQ ID N° 6, SEQ ID N° 16 ou SEQ ID N° 17**,après alignement optimal. Par « polypeptide dont la séquence d'acides aminés présente un pourcentage d'homologie d'au moins 80%, de préférence d'au moins 85%, d'au moins 90%, d'au moins 95%, d'au moins 98% et préférentiellement d'au moins 99% après alignement optimal avec une séquence de référence », on entend désigner les polypeptides présentant certaines modifications par rapport au polypeptide de référence, comme en particulier une ou plusieurs délétions, troncations, un allongement, une fusion chimérique, et/ou une ou plusieurs substitutions.

Par pourcentage d'homologie entre deux séquences d'acides aminés au sens de la présente invention, on entend désigner le pourcentage de résidus d'acides aminés similaires (identiques ou substitution conservative) ou, de préférence, identiques entre les deux séquences à comparer, obtenu après alignement optimal des deux séquences, les différences entre les deux séquences pouvant être réparties au hasard et le long de la séquence. On entend désigner par "alignement optimal", l'alignement pour lequel le pourcentage d'homologie déterminé comme ci-après est le plus élevé. L'alignement optimal qui correspond à l'alignement qui donne le nombre le plus important d'acides aminés identiques après alignement entre les deux séquences d'acides aminés comparées, peut être obtenu, éventuellement par glissement des séquences les unes par rapport à l'autre et/ou l'insertion dans l'une des séquences comparées, d'un gap entre 2 acides aminés consécutifs.

Le degré d'homologie peut, au sens de l'invention, être déterminé localement par les techniques de comparaison de séquences protéiques, et par exemple la technique BLAST P telle que décrite par ALTSCHUL S.F. et al. dans Nucleic Acid Research 1997, 25, 3389-3402. Dans ce cas, est considérée comme une séquence protéique, homologue d'une autre séquence protéique de référence, une protéine qui présente, sur un nombre d'acides aminés au moins égal à 80% du nombre d'acides aminés de la séquence protéique de référence, de préférence sur la longueur totale de la séquence de référence, un pourcentage d'homologie correspondant aux valeurs indiquées précédemment, à savoir au moins 80% d'homologie, de préférence au moins 85%, au moins 90%, au moins 95%, au moins 98% et préférentiellement au moins 99% d'homologie. Le pourcentage d'homologie peut être calculé selon donné par la technique BLAST P version 2.2.13. De façon préférée, la comparaison est effectuée sur la totalité de la séquence du polypeptide à comparer et la séquence complète du polypeptide de référence et le degré d'homologie d'au moins 80%, de préférence d'au moins 85%, d'au moins 90%, d'au moins 95%, d'au moins 98% et préférentiellement d'au moins 99% d'homologie, doit être obtenu en comparant la totalité de la séquence de la protéine à comparer et la séquence complète de la protéine de référence.

De façon préférée, le pourcentage d'homologie est calculé, de façon manuelle, sur la longueur totale du polypeptide dont on souhaite déterminer le degré d'homologie avec un polypeptide de référence. Le pourcentage d'homologie est alors calculé, après alignement optimal, en comptant le nombre de positions pour lesquellesles polypeptides présentent des acides aminés identiques et en divisant ce nombre de positions identiques par la longueur de la séquence du polypeptide le plus long et en multipliant le résultat obtenu par 100 pour obtenir le pourcentage d'homologie entre ces deux séquences.

La présente invention a également pour objet tout acide nucléique purifié ou isolé constitué par un enchaînement de nucléotides codant pour les polypeptides selon l'invention. Il peut s'agir de séquences d'origine naturelle ou artificielle, et notamment d'ADN génomique, d'ADNc, d'ARNm, de séquence hybrides ou de séquences synthétiques ou semi-synthétiques. Il peut être obtenu par toute technique connue de l'homme du métier, et notamment par criblage de banques, par synthèse chimique ou encore par des méthodes mixtes incluant la modification chimique ou enzymatique de séquences obtenues par criblage de banques.

Par acide nucléique, séquence nucléique ou d'acide nucléique, polynucléotide, oligonucléotide, séquence de polynucléotide, séquence nucléotidique, termes qui seront employés indifféremment dans la présente description, on entend désigner un enchaînement précis de nucléotides, modifiés ou non, permettant de définir un fragment ou une région d'un acide nucléique, comportant ou non des nucléotides non naturels, et pouvant correspondre aussi bien à un ADN double brin, un ADN simple brin que des produits de transcription desdits ADN, et/ou un fragment d'ARN.

Les acides nucléiques selon l'invention peuvent notamment être préparés par synthèse chimique et génie génétique en utilisant les techniques bien connues de l'homme du métier et décrites, par exemple, dans SAMBROOK et al. "Molecular Cloning : a Laboratory Manual" publié en 1989 par Cold Spring Harbor Press, NY, 2ème édition.

Par exemple, la synthèse des séquences d'ADNc selon l'invention peut être effectuée par amplification des ARNm à l'aide de la méthode PCR (de l'anglais "Polymerase Chain Reaction"), comme décrit, par exemple, par GOBLET et al. (Nucleic Acid Research, 17, 2144, 1989) en utilisant des oligonucléotides synthétiques comme amorces, définis à partir de la séquence d'ADN de GRAS.

Le fragment d'acides nucléiques amplifié peut ensuite être cloné selon les techniques décrites dans AUSUBEL et al. (Current Protocols in Molecular Biology, Green Publishing Associates and Wiley Interscience, New-York, 1989, Mises à jour jusqu'en 1997, chapter 3).

Les protéines et fragments peptidiques selon l'invention peuvent être obtenus par la technique du génie génétique qui comprend les étapes de :
- culture d'un micro-organisme transformé ou de cellules eucaryotes ou procaryotes transformées à l'aide d'un vecteur d'expression portant une séquence d'acides nucléiques selon l'invention ; et
- récupération de la protéine ou du fragment peptidique produit par le dit micro-organisme ou lesdites cellules eucaryotes.

Cette technique est bien connue de l'homme du métier. Pour plus de détails la concernant, on pourra se référer à l'ouvrage ci-après : Recombinant DNA Technology I, Editors Ales Prokop, Raskesh K Bajpai ; Annals of the New-York Academy of Sciences, volume 646, 1991.

Ils peuvent également être préparés par les synthèses peptidiques classiques bien connues de l'homme du métier.

L'invention a également pour objet des micro-organismes procaryotes et les cellules eucaryotes transformés à l'aide d'un vecteur d'expression contenant une séquence d'ADN selon l'invention. Ce vecteur d'expression, qui peut être, par exemple, sous la forme d'un plasmide, cosmide ou phage, doit comporter, outre la séquence d'ADN de l'invention, les moyens nécessaires à son expression, tels que notamment un promoteur, un terminateur de transcription, une origine de réplication et de préférence un marqueur de sélection. La transformation des micro-organismes et des cellules eucaryotes est une technique bien connue de l'homme du métier qui pourra aisément déterminer, en fonction du micro-organisme à transformer, les moyens nécessaires à l'expression de la séquence d'ADN selon l'invention.

Comme micro-organisme procaryote, on peut citer E. coli et comme eucaryote la levure Saccharomyces cerevisiae.

A titre d'exemples de cellules eucaryotes qui conviennent aux fins de l'invention, on peut citer notamment les cellules de singe COS, les cellules d'ovaire de hamster chinois CHO, les cellules d'insectes Sf9, la lignée de lymphome T humain Jurkat, etc., toutes étant répertoriées à l'ATCC.

De manière avantageuse, on pourra utiliser un système mettant en oeuvre un vecteur de type baculovirus. Ce système permet de produire des protéines étrangères aux cellules (cellules Sf9) d'un insecte (Spodoptera Frugiperda) en utilisant une recombinaison *in vivo* entre un vecteur de transfert (plasmide) qui contient la séquence d'ADN étrangère, d'une part et d'autre part, le génome d'un virus. (O'Reilly, D., L.K. Miller, and V.A. Luckow. 1992. Baculovirus expression vectors: A Laboratory manual. W.H. Freeman and Compan, New York, Y.)

L'invention a également pour objet les micro-organismes procaryotes et les cellules eucaryotes cotransformées avec des vecteurs d'expression contenant la séquence d'ADN codant pour un polypeptide selon l'invention, lesdits vecteurs d'expression contenant de plus des moyens utiles à leur expression, y compris dans le système double-hybride en levure.

Les polypeptides selon l'invention sont particulièrement intéressants sur le plan thérapeutique. Leur activité thérapeutique est liée plus particulièrement à leur aptitude à entraîner la migration des cellules dendritiques à partir de tissus ou muqueuses, qui a été mise en évidence *in vitro* et *in vivo* chez la souris. Ils seront en particulier utiles pour le traitement des maladies où il est nécessaire d'obtenir une déplétion en cellules dendritiques d'un tissu périphérique, telles que la peau ou les muqueuses.

L'invention concerne également les polypeptides et les protéines de fusion selon l'invention pour leur utilisation en tant que médicament ainsi que les compositions pharmaceutiques contenant l'un des polypeptides ou l'une des protéines de fusion selon l'invention, en association avec au moins un excipient pharmaceutique convenable. Les polypeptides selon l'invéntion peuvent également être utilisés en fusion, par exemple avec une autre protéine telle que la GST de séquence **SEQ ID N°7 :**

S P I L G Y W K I K G L V Q P T R L L L E Y L E E K Y E E H L Y E R D E G D K W R
N K K F E L G L E F P N L P Y Y I D G D V K L T Q S M A I I R Y I A D K H N M L G G C
P K E R A E I S M L E G A V L D I R Y G V S R I A Y S K D F E T L K V D F L S K L P E M
L K M F E D R L C H K T Y L N G D H V T H P D F M L Y D A L D V V L Y M D P M C L D
A F P K L V C F K K R I E A I P Q I D K Y L K S S K Y I A W P L Q G W Q A T F G G G D
H P P K S D L

Ces compositions pharmaceutiques sont préparées selon les techniques classiques bien connues de l'homme de l'art. Les excipients pharmaceutiques sont choisis selon la forme pharmaceutique et le mode d'administration souhaité, par exemple, administration orale, sublinguale, sous-cutanée, intramusculaire, intra-veineuse, topique ... Les compositions de forme adaptée à l'application topique, par exemple de type crème ou onguent, sont préférées.

La présente invention a aussi pour objet l'utilisation d'un polypeptide ou d'une protéine de fusion selon l'invention pour la préparation d'un médicament destiné à prévenir ou traiter des pathologies de la peau ou des muqueuses impliquant des cellules dendritiques ou des cellules de Langerhans. En particulier, un polypeptide selon l'invention pourra être utilisé pour la fabrication d'un médicament destiné à prévenir ou traiter des pathologies choisies parmi : la dermatite atopique, les inflammations, les maladies auto-immunes, le Lupus érythémateux disséminé, la polyarthrite, le psoriasis, la réaction du greffon contre l'hôte, le rejet de greffe, ou les allergies, telles que les allergies de contact, par exemple au latex, aux cosmétiques, aux parfums, aux tatouages, aux métaux et à leurs sels, les phénomènes allergiques pulmonaires dues aux allergènes aériens, les rhinites allergiques, l'asthme ou les allergies alimentaires. Selon les modalités d'utilisation et les cellules dendritiques utilisées comme cible, l'administration d'un polypeptide selon l'invention peut également entraîner un renforcement de la réponse immune.

L'invention va maintenant être décrite en détails à l'aide de l'exposé expérimental ci-après.

Une grande partie des techniques décrites dans ces exemples, bien connues de l'homme du métier, est exposée en détail dans l'ouvrage de SAMBROOK *et al. (supra)* ou dans l'ouvrage de AUSUBEL *et al. (supra).*

La description ci-après sera mieux comprise à l'aide des **fig. 1** à **9** sur lesquelles :
- la **fig. 1** représente l'alignement des séquences d'ADN codant pour la protéine GRA5, chez différentes souches de *Toxoplasma gondii,*
- la **fig. 2** représente l'alignement des séquences peptidiques de la protéine GRAS, chez différentes souches de *Toxoplasma gondii,*
- les **fig.3** à **7** illustrent les indexes de migration obtenus dans différents tests effectués.
- La **fig. 8** présente les activités de déclenchement de migration *in vitro* du peptide de **SEQ ID N°16** et du peptide de **SEQ ID N°17,** en fonction de leur concentration.
- la **fig.9** montre le nombre de cellules de Langerhans et de cellules dendritiques repéré par un marquage avec un anticorps anti-CD11c, obtenu sur un test *in vitro* chez la souris.

### EXEMPLES

### Réactifs utilisés :

- Milieu de culture RPMI 1640 avec Glutamax (réf 61870-010, In Vitrogen Gibco, Grand Island, NY, USA)
- Sérum de bovin nouveau né (SVF, réf 16000-036, In Vitrogen Gibco, Grand Island, NY, USA), décomplémenter le SVF par chauffage de 30 minutes à 56°C
- Albumine sérique bovine (réf 652 237, 100 mg/ml, Roche Diagnostics GmbH, Mannheim RFA)
- 2-mercaptoéthanol (réf 31 350-010, 50 mM, In Vitrogen Gibco, Grand Island, NY, USA)
- Gentamicine (réf 15 710-049, 10 mg/ml, In Vitrogen Gibco, Grand Island, NY, USA)
- Penicillin-streptomycin (réf 15140-122 pénicilline : 10 000 U/ml, : streptomycine 10 000 U/ml In Vitrogen Gibco Grand Island, NY, USA)
- GM-CSF (de l'anglais "granulocyte macrophage colony-stimulating factor recombinant"; réf 11343128, 1 mg, 11 x 10⁶ U/mg, AL-Immunotools, Friesoythe, Germany)
- TNF-α (de l'anglais "recombinant human tumour necrosis factor"; activité spécifique 2 x 10⁷ U/ml, 200 µg/ml; Genzyme Corp., Boston, MA, USA) Boehringer
- TGF-β1 (transforming growth factor béta 1, 10 µg; réf 100B010; R&D Systems, Minneapolis, MN, USA)
- MIP-3β (de l'anglais "macrophage inflammatory protein 3β∀, réf 361-MI, 25 µg/ml; R&D Systems, Minneapolis, MN, USA)
- Bleu Trypan (réf T8154, 0,4 %; Sigma-Aldrich, Saint-Louis, MO, USA)
- Matrigel GFR (de l'anglais "Matrigel Growth Factor Reduced", réf 354263 ,5 ml; BD Biosciences, San Jose, CA, USA).
- Plaque de culture 6 puits (réf [35]3502, Falcon)
- Plaque de culture 12 puits (réf [35]3503, Falcon)
- Plaque de culture 24 puits (réf [35]3504, Falcon)
- Insert de porosité 8 µm pour plaque 6 puits (réf [35]3093, Falcon)
- Insert de porosité 8 µm pour plaque 12 puits (réf [35]3082, Falcon)
- Insert de porosité 8 µm pour plaque 24 puits (réf [35]3097, Falcon)
- Tubes de culture de 5 ml à fond rond (réf [35]2054, Falcon)
- Cellules de numération jetables Kovaslide (réf 87144, Hycor Biomedical LTD, Penicuik, United Kingdom)
- Enzymes de restriction et de modification (Boehringer-Mannheim, Mannheim, Germany).
- T4 DNA ligase and Taq-Polymerase (Promega, Charbonnières, France).
- Souche bactérienne BL21 (Stratagene).
- Vecteurs plasmidiques : pPCR Amp Script SK(+) (cloning kit, Stratagene, La Jolla, CA); pGEX -3X (Pharmacia , Upsala, Suède)
- Billes d'agarose couplées au Glutathion et le glutathion réduit (SIGMA Chimie, St-Quentin, France).
- Kit Bradfort (Bio-Rad)
- Kit "Deaza G/A^{T7} sequencing^{™} Mixes" (Pharmacia)
- Kit "AutoRead^{™} Sequencing Kit" (Pharmacia).
- Oligonucléotides

| **Amorce** | **Séquence** | **Sens^{a}** | **Site de restriction créé** |
|---|---|---|---|
| 21.19 | SEQ ID N°8 5'-TCAA**CTCGAG**CCGCGTCGGTTTGGTTTGTGC-3' | S | ***XhoI*** |
| 21.20 | SEQ ID N°9 5'-GTAT**CTCGAG**GGGCAGACGTGGCCGGTTTCC-3' | AS | ***Xho*I** |
| G5N5' | SEQ ID N°10 5'-GT**CCCGGGG**GTTCAACGCGTGACGTAG-3' | S | ***Sma*I** |
| G5N5'76K | SEQ ID N°11 5'-G**TCCCGGGG**GTTCAACGTGTGACACAG-3' | S | ***Sma*I** |
| G5N3' | SEQ ID N°12 5'-CC**GAATTCCCT**CACTGGATGTCCAG-3' | AS | ***Eco*RI** |

| | | | |
|---|---|---|---|
| ^{a} S : sens, AS : anti-sens | | | |

### Solutions utilisées :

### - Milieu RPMI 5%SVF ou 10%SVF

Dans un flacon de 500 ml de milieu, ajouter 25 ou 50 ml de SVF. Le jour d'utilisation prélever stérilement le volume nécessaire, ajouter 10 µl de gentamycine par ml de milieu et amener à température ambiante avant utilisation.

### - Milieu RPMI 1% BSA

Dans un flacon de 100 ml, ajouter 1 ml de BSA à 99 ml de RPMI. Le jour d'utilisation prélever stérilement le volume nécessaire, ajouter 10 µl de gentamycine par ml de milieu et amener à température ambiante avant utilisation.

### - Solution de Matrigel

Préparer le Matrigel à 4°C sur la glace avec du milieu RPMI à 4°C. Le Matrigel est dilué avec du milieu RPMI de façon à obtenir une concentration de 8,35 mg/ml, il est ensuite réparti par fractions aliquotes de 100 µl et conservé à -20 °C.

### - Milieu RPMI/chimiokine

Ajouter 100 µl de MIP3β à 5 ml de RPMI 1% BSA, homogénéiser au vortex. Cette solution permet de préparer 2 puits de plaque P6, 5 puits de plaque P12, 12 puits de plaque P24.

### - Solution de GM-CSF

Une solution mère est préparée en ajoutant 5,5 ml d'eau stérile à 1 mg de GM-CSF ; cette solution est à 2 x 10⁶ U/ml. Elle est répartie par fractions aliquotes de 500 µl et conservée à -80°C.

A partir de la solution précédente on prépare une solution secondaire par addition d'un tube de 500 µl dans 4,5 ml de RPMI 5%SVF. Cette solution est à son tour répartie par fractions aliquotes de 100 µl et conservée à -80°C.

Un tube décongelé se garde 8 jours à 4°C, on utilise 1 µl de cette solution par ml de milieu de culture. - Solution de TNF-α

Le TNFα est réparti par 100 µl et conservé à -80°C. Une solution mère est préparée en diluant un tube de 100 µl par addition de 700 µl de RPMI 10% SVF. Cette solution est conservé en fractions aliquotes de 100 µl à -80°C.

A partir de la solution précédente on prépare une solution secondaire par addition d'un tube de 100 µl dans 0,9 ml de RPMI 5% SVF. Cette solution est à son tour répartie par fractions aliquotes de 15 et 30 µl et conservée à -80°C.

Un tube décongelé se garde 8 jours à 4°C, on utilise 1 µl de cette solution par ml de milieu de culture.

### - Préparation du TGF-β1

Les 10 µg contenus dans le flacon sont remis en suspension dans 5 ml de RPMI 10% SVF en présence de 20 µl d'HCl 1N. Cette solution mère est conservé en fractions aliquotes de 250 µl à -80 °C.

Une solution intermédiaire est préparée en diluant un tube de 250 µl dans 750 µl de RPMI 10% SVF. Cette solution est répartie en fractions aliquotes de 15 et 30 µl et conservée à -80°C.

Un tube décongelé se garde 8 jours à 4°C, on utilise 1 µl de cette solution par ml de milieu de culture.

### Stabilité des réactifs et solutions

Le SVF, le Matrigel et les cytokines sont conservées congelées à -80°C sans limite de durée.

Le milieu de culture, l'albumine sérique bovine, le 2-mercaptoéthanol, la gentamicine et le bleu Trypan sont conservés au réfrigérateur jusqu' à date de péremption.

Les milieux avec sérum sont conservés au réfrigérateur et utilisés dans le mois. Les solutions intermédiaires de cytokine et de Matrigel se conservent à -80°C après répartition en fractions aliquotes.

### Culture des tachyzoïtes de la souche RH sur la lignée THP1

1. Les cellules THP1 sont lavées en RPMI (10 min à 1400 t/min) puis comptées en présence de bleu Trypan (v/v).
2. Dans un flacon de 25 cm² ajouter 10 x 10⁶ THP1 et 10 x 10⁶ tachyzoïtes. La culture s'effectue dans 10 ml de RPMI à 5% de SVF et avec 1% de penicillin-streptomycin.
3. A 24h les cellules peuvent être congelées. Sinon elles sont transférées dans un flacon de 75 cm² en ajoutant 10 ml de milieu.
4. A 48h les cellules doivent être complètement lysées, et les tachyzoïtes sont récupérés.
5. Récupérer les tachyzoïtes avec une pipette de 25 ml, dissocier les amas par aspiration et refoulement en plaquant le bas de la pipette sur le fond du flacon. Lyser les THP1 restantes par transfert avec une seringue à insuline équipée de son aiguille. Passer en tubes de 50 ml (40 ml par tube).
6. Centrifuger à basse vitesse (4 min à 700 rpm), récupérer le surnageant qui contient les tachyzoïtes.
7. Si la présence de débris ou de cellules THP1 intactes est trop importante, filtrer le surnageant sur filtre 8 µM puis 3 µM pour isoler les tachyzoïtes des THP1 survivantes.
8. Compléter avec du PBS et centrifuger 20 min à 1 400 rpm.
9. Reprendre le culot avec 10 ml de PBS en agitant au vortex, ne pas dépasser le réglage de 1 500.
10. Effectuer le comptage des tachyzoïtes.

### Culture des tachyzoïtes de la souche PRU sur la lignée THP1

1. Dans un flacon de 25 cm² ajouter 10 x 10⁶ THP1 et 30 x 10⁶ tachyzoïtes. La culture s'effectue dans 10 ml de RPMI à 5% de SVF et avec 1% de penicillin-streptomycin.
2. A 24h transférer dans un flacon de 75 cm² en ajoutant 10 ml de milieu.
3. Changer le milieu de culture tous les 3-4 jours par centrifugation 1400 t/min, 10 min. Dédoubler la boîte si les THP1 se sont multipliées et si le milieu est jaune.
4. Périodiquement vérifier le taux d'infection par marquage des THP1 par acridine orange et BET (5 µg/ml de chaque). Lorsque le pourcentage de cellules infectées atteint 30% il est possible de congeler.
5. Poursuivre la culture jusqu'à obtenir un maximum de tachyzoïtes libres et une lyse complète des cellules.
6. Récupérer les tachyzoïtes avec une pipette de 10 ml et passer en tubes de 50 ml (40 ml par tube).
7. Centrifuger à basse vitesse (4 min à 700 rpm), récupérer le surnageant qui contient les tachyzoïtes.
8. Si la présence de débris ou de cellules THP1 intactes est trop importante, filtrer le surnageant sur filtre 8 µM puis 3 µM pour isoler les tachyzoïtes des THP1 survivantes. Passer au préalable 2 ml de PBS puis les tachyzoïtes, ne pas utiliser de piston et laisser la suspension s'écouler par gravité.
9. Compléter avec du PBS et centrifuger 20 min à 1 400 rpm.
10. Reprendre le culot avec 10 ml de PBS en agitant au vortex, ne pas dépasser le réglage de 1 800.
11. Effectuer le comptage des tachyzoïtes.

### Préparation des antigènes secretés (ESA)

Les ESA sont obtenus à partir de tachyzoïtes de différentes souches. Les tachyzoïtes sont récupérés après passage en culture sur cellules THP1 à partir : d'une ascite de souris (souche virulente RH); du cerveau d'une souris (souche moins virulente Pru); de cellules parasitées congelées dans l'azote liquide.
1. Centrifuger les tachyzoïtes 20 min à 1 400 rpm
2. Reprendre le culot dans du milieu RPMI avec 10% de SVF ou 2 mg/ml d'albumine humaine purifiée pour avoir une concentration de 150 x 10⁶ tachyzoïtes par ml.
3. Verser soit dans un puits de plaque à 6 puits soit dans un tube de culture. Incuber 3 heures à 37°C. Agiter la plaque toutes les 15 min, le tube est placé sur l'agitateur rotatif Dynal dans l'étuve.
4. Après 3 h, transférer dans un tube Eppendorf et centrifuger 15 min à 1 000 rpm.
5. Récupérer le surnageant, filtrer sur 0,22 µ et congeler dans l'azote liquide.
6. Récupérer le culot et vérifier que les parasites sont toujours vivants.

### Préparation de l'ADN génomique

Les préparations d'ADN génomique des souches de *T. gondii* (RH, Pru, 76K, CEP et C56) ont été réalisées à partir de tachyzoïtes provenant d'ascites de souris infestées ou issus de l'infection d'un tapis de cellules Hep-II. Les parasites ont été recueillis puis purifiés après filtration sur une membrane de polycarbonate d'une porosité de 3 µm (Nucléopore). Après une centrifugation de 10 minutes à 3000 rpm, les parasites ont été lavés deux fois en PBS. Le culot a été repris dans une solution d'EDTA 0,1 M, Tris-HCl 10 mM pH 8,0, SDS 1 % et protéinase K 2 µg/ml et incubé une nuit à 50°C. Les protéines contaminantes ont été éliminées par une extraction au phénol/chloroforme. L'ADN a été précipité avec deux volumes d'éthanol absolu puis lavé en éthanol 70 %. L'ADN a été solubilisé dans un tampon TE (Tris-HCl 10 mM, EDTA 1 mM, pH 8,0) pendant toute une nuit à 4°C ; sa concentration a été déduite après estimation de la densité optique de la solution à 260 nm.

### Amplification génique (PCR)

Les réactions d'amplification PCR ont été réalisées dans des conditions standards à l'aide de l'appareil Peltier Thermal Cycler (PTC-200, MJ Research) à partir de 5 à 10 ng de matrice plasmidique ou de 1 µg d'ADN génomique, dans un volume réactionnel de 50 µl. Les matrices ont été placées en présence de 0,5 µM d'amorces oligonucléotidiques, d'un mélange de désoxyribonucléotides (dATP, dCTP, dGTP, dTTP, 200 µM, Pharmacia) et de 4 unités de l'ADN polymérase Deep Vent. Les cycles sont 94°C - 5 min (94°C 1min - 60°C 1 min - 72°C 2 min) 35 fois, et avec une extension finale de 7 min à 72°C.

### Sequençage

Le séquençage manuel a été réalisé en présence de [³⁵S] α-dATP (Amersham) à l'aide du kit "Deaza G/A^{T7} sequencing ^{™} Mixes" (Pharmacia). Les réactions de séquence ont été analysées après migration électrophorétique sur un gel de polyacrylamide 6 % en tampon TBE 1X. Le gel a ensuite été fixé 15 minutes dans une solution d'acide acétique 10%, méthanol 10% puis séché et autoradiographié (film Biomax, Kodak).

Le séquençage automatique a été réalisé en présence de fluorochrome couplé au dATP (Cy^{™}5-13-dATP) à l'aide du kit "AutoRead^{™} Sequencing Kit" (Pharmacia).

### Production des protéines recombinantes en fusion avec glutathione S-transferase (GST)

Les polypeptides de fusion ou la protéine GST sauvage ont été produits en suivant les procédures recommandées par Pharmacie. Brièvement, à partir d'une culture de BL21 recombinantes en phase exponentielle, l'expression a été induite pendant 3h à 37°C par ajout de isopropyl-β-D-thiogalactoside (IPTG, Roche) à 1 mM. Les cellules sont centrifugées à 4000 × g pendant 15 min et le culot est resuspendu dans un tampon de lyse (phosphate 0,02 M pH 7,4, phényl méthyl sulfonyl fluoride 0,5 mM, EDTA 1 mM, Triton X100 1%). Les cellules sont lysées sur la glace par deux passages successifs à la presse de French (1000 psi). Le lysat est centrifugé à 10 000 × g pendant 10 min à 4 °C. Les polypeptides de fusion sont purifiés à partir du surnageant par chromatographie d'affinité en utilisant du gluthation immobilisé sur billes d'agarose. L'élution des protéines de fusion est réalisé par incubation des billes dans une solution glutathion réduit 5mM, Tris HCl 50 mM (pH 8,0). Les protéines recombinantes éluées sont concentrées et dialysées dans un tampon phosphate 0,1M pH 7,4. La qualité des protéines purifiées a été vérifiée sur un gel SDS-PAGE et la concentration des protéines a été déterminée par le test de Bradfort (kit Bio-Rad).

Les gènes natifs de la protéine GRA5 issue de différentes souches ont été clonés dans un vecteur pGEX 3X contenant le site de clivage du facteur Xa entre la GST et la protéine d'intérêt.

Le polypeptide de **SEQ ID N°3** (fragment de GRA5-RH) (sans la thréonine C-terminale) en fusion avec la GST possède la séquence suivante :

SEQ ID N°13 :
S P I L G Y W K I K G L V Q P T R L L L E Y L E E K Y E E H L Y E R D E G D K W R N K
K F E L G L E F P N L P Y Y I D G D V K L T Q S M A I I R Y I A D K H N M L G G C P K
E R A E I S M L E G A V L D I R Y G V S R I A Y S K D F E T L K V D F L S K L P E M L K
M F E D R L C H K T Y L N G D H V T H P D F M L Y D A L D V V L Y M D P M C L D A F
P K L V C F K K R I E A I P Q I D K Y L K S S K Y I A W P L Q G W Q A T F G G G D H P
P K S D L I E G R↓G I P G G S T R D V G S G G D D S E G A R G R E Q Q V Q Q H
E Q N E D R S L F E R G R A A V T G H P V R E F I V T D

La séquence de la GST apparaît avant le site de clivage (↓), entre le site de reconnaissance du Facteur Xa, représenté en gras souligné et le polypeptide issu de la protéine GRA5-RH (en gras), lui-même encadré par les acides aminés créés par le polylinker du site d'insertion, représentés en italique.

Le polypeptide de **SEQ ID N°5** (fragment de GRA5-76K) (sans la thréonine C-terminale) en fusion avec la GST possède la séquence suivante :

SEQ ID N°14 :
S P I L G Y W K I K G L V Q P T R L L L E Y L E E K Y E E H L Y E R D E G D K W R N K
K F E L G L E F P N L P Y Y I D G D V K L T Q S M A I I R Y I A D K H N M L G G C P K

ERAEISMLEGAVLDIRYGVSRIAYSKDFETLKVDFLSKLPEMLK
MFEDRLCHKTYLNGDHVTHPDFMLYDALDVVLYMDPMCLDAF
PKLVCFKKRIEAIPQIDKYLKSSKYIAWPLQGWQATFGGGDHP
PKSDLIEGR↓GIPGGSTCDTGSGGDDSEGAWGGEQQQVQQ
HGQSEDRSLFERGRAAVTGHPVREFIVTD

Le polypeptide de SE ID N°6 (fragment de GRA5-CEP ou de GRA-C56) (sans la thréonine C-terminale) en fusion avec la GST possède la séquence suivante :

SEQ ID N°15 :
S P I L G Y W K I K G L V Q P T R L L L E Y L E E K Y E E H L Y E R D E G D K W R N K
K F E L G L E F P N L P Y Y I D G D V K L T Q S M A I I R Y I A D K H N M L G G C P K
E R A E I S M L E G A V L D I R Y G V S R I A Y S K D F E T L K V D F L S K L P E M L K
M F E D R L C H K T Y L N G D H V T H P D F M L Y D A L D V V L Y M D P M C L D A F
P K L V C F K K R I E A I P Q I D K Y L K S S K Y I A W P L Q G W Q A T F G G G D H P
P K S D L I E G R↓G I P G G S T R D V G S G A D D S E G A G G R E R Q Q V Q Q H
E Q N E D R S L F E R G R A A V T G H P V R E F I V T D

### Analyse du polymorphisme des gènes GRAS (souches RH, 76K Pru, CEP et C56)

Le couple d'amorces oligonucléotidiques (21.19 et 21.20) a été utilisé pour amplifier, par PCR, les fragments d'ADN génomique correspondant au gène *GRA5* (Lecordier et al., Mol. Biochem. Parasitol. 1993, 59, 143-154) à partir de l'ADN de chaque souche parasitaire. Les fragments amplifiés possèdent la région 5' non traduite (notée 5' UT pour "untranslated" : non traduite), le cadre de lecture ouvert et la région 3' non traduite (3' UT) du gène GRAS. La taille des fragments amplifiés (∼0,95 kb) a été vérifiée sur un gel d'agarose 1%. Après purification, les fragments ont été clonés dans le vecteur PCR-Script SK(+) digéré par SfrI. Les fragments clonés ont été séquencés dans les deux sens. Les séquences obtenues ont été alignées avec la séquence connue de la souche RH européenne de génotype 1 (Lecordier *et al.* 1993 *supra ;* GenBank numéro d'accession : L06091) (**Figures 1** et **2**). Sur la **Figure 2**, les séquences en acides aminés déduites du gène GRAS obtenues chez les souches 76K et Pru pour le groupe II et chez les souches CEP et C56 pour le groupe III ont été alignées à celle de la souche RH du groupe I. Les acides aminés identiques à ceux de la souche RH prise comme référence ont été notés "-". Les acides aminés substitués sont indiqués, les substitutions non conservatives sont indiquée en caractère gras. Les acides aminés correspondant aux deux régions hydrophobes (peptide signal et domaine transmembranaire), qui encadrent les acides aminés de la région N-terminale qui a été testée, sont indiqués en italique.

L'analyse comparative des séquences codantes pour *GRA5* obtenues à partir des souches des trois types (I, II, III) a révélé :
1. une très grande conservation de la séquence à l'intérieur d'un même groupe avec 99,72 % d'homologie entre les souches 76K et Pru pour le groupe II et 100 % d'homologie entre les souches CEP et C56 pour le groupe III ;
2. une homologie plus importante entre les souches des groupes I et III (supérieure à 99 %) qu'entre les souches des groupes I et II (environ 98 %);
3. toutes les substitutions à l'exception de la dernière guanine pour les souches du groupe II conduisent à une modification de la séquence protéique primaire avec six acides aminés différents dans la souche Pru (groupe II) et trois dans les souches du groupe III (**Figure 2**) ;
4. la présence d'une région polymorphique dans la région N-terminale, après le site de clivage potentiel du peptide signal la plupart des acides aminés substitués est présente dans la région N terminale de la protéine, à l'exception d'une substitution dans les souches du groupe II, où la lysine (K94) a été remplacée par une arginine (R);
5. sur les six substitutions observées dans la séquence des protéines GRA5 de type II, trois sont conservatives et trois autres concernent des acides aminés chargés remplacés par des acides aminés hydrophobes dans la séquence de type II (R42 / W42 ; R44 / G44 ; E53 / G53) ;
6. sur trois substitutions observées dans la séquence des protéines GRAS de type III, deux sont conservatives, la troisième correspond également au remplacement un acide aminé chargé par un hydrophobe (R42 / G42).

### Expression et purification des variants de GRAS (région N-terminale hydrophiles) sous forme recombinante en fusion avec la GST:

Les variants type I, II and III de la région N-terminale hydrophile de GRAS ont été exprimés sous forme de polypeptides recombinants fusionnés à la glutathione S-transferase (GST) en utilisant le vecteur d'expression pGEX-3X. Pour ce faire, les plasmides d'expression pTg GRA5-Nt type I, pTg GRA5-Nt type II and pTg GRAS-Nt type III ont été construits. Les séquences d'ADN utilisées pour l'expression des variants GRA5 Nt types I, II and III sont celles de GRAS RH, 76K CEP et C56 (**Figure 1**). Le fragment de 150 paires de bases (pb) codant pour la partie N-terminale de GRAS a été amplifiés en utilisant le couple d'amorces G5N5' (ou G5N5'76K) et G5N3'. Les fragments amplifiés ont été digérés par *Sma*I et *EcoR*I et inséré par ligation aux sites *Sma*I /*EcoR*I du vecteur pGEX 3X, en phase avec la séquence codant pour la glutathione S-transférase (GST). Les constructions ont été vérifiées par séquençage.

Pour l'expression des protéines recombinantes, des cellules compétentes BL 21 ont été transformées par l'ADN plasmidique parental (pGEX-3X) ou recombinant (GRAS-Nt type I, pTg GRAS-Nt type II, pTg GRAS-Nt type III). Après induction de l'expression suivie de la purification, les protéines de fusion ont été analysées par SDS-PAGE. Le rendement de production pour la GST est de 25 mg/ litre de culture et de 40 à 50 mg/litre de culture pour les trois protéines de fusion (GST-GRAS-Nt type I, GST-GRA5-Nt type II, GST-GRA5-Nt type III).

### TEST SUR LA MIGRATION DES CELLULES DENDRITIQUES IN VITRO

### Culture des cellules dendritiques, cellules de Langerhans

Les précurseurs CD34 isolés de sang du cordon ombilical sont différenciés en cellules dendritiques comme il est indiqué dans les références suivantes : Rougier et a/., J. Invest. Dermatol. 1998, 110, pp 348-352 et Eur. J. Cell. Biol. 1998, 75 pp 287-293. Après 4 à 6 jours de différenciation, les cellules obtenues sont congelées dans l'azote liquide et peuvent être conservées plusieurs années.

### Test de migration

Le test utilisé est décrit dans les deux publications Staquet et al. (Int Arch Allergy Immunol. 2004, 133, 348-56), et Diana *et al.* 2004 citée *supra.* Des plaques avec ou sans Matrigel peuvent être utilisés, les résultats sont comparables.
1. Les cellules dendritiques sont décongelées et mises en culture en milieu RPMI à 5% de SVF avec de la gentamycine, GM-CSF et TNFα pour obtenir des cellules dendritiques interstitielles. Pour obtenir des cellules de Langerhans, on utilise le milieu avec du GM-CSF et du TGFβ1. Les deux types de cellules fonctionnent de manière équivalente dans le test de migration.
2. 48h plus tard, les cellules sont comptées et réparties par lots de 300 000 dans des tubes de culture Falcon sous un volume de 300 µl. On ajoute les activateurs: base de Bandrowski (1,27 ng/ml en dilution finale), LPS (25 ng/ml en dilution finale) ou les ESA à une dilution finale du 1/20^{e}. Les tubes sont remis en culture.
3. Si la migration va s'effectuer en présence de Matrigel, les inserts avec le Matrigels sont traités comme suit : ajouter 735 µl de milieu RPMI dans le tube de matrigel sorti du congélateur et décongeler rapidement par des allers et retours à la pipette. Ensuite ajouter un volume égal de milieu RPMI, mélanger doucement pour éviter de faire des bulles, la concentration en matrigel est de 0,5 mg/ml. Ces 2 ml de cette solution permettent de préparer 4 inserts de plaque P6, 19 inserts de plaque P12, 66 inserts de plaque P24.
4. Positionner les inserts dans leur plaque respective. Les dépôts se font doucement, à l'aide d'une micropipette, en évitant de faire des bulles. Déposer 30 µl sur chaque insert d'une plaque 24 puits, 100 µl sur chaque insert d'une plaque 12 puits et 420 µl sur chaque insert d'une plaque 6 puits. Laisser évaporer sous la hotte, entre 4 et 20 heures. Avant de déposer la suspension cellulaire, rincer chaque insert 2 fois en RPMI
5. Après 24 h, les tubes sont récupérés, les cellules dénombrées et remises en suspension dans le milieu de culture à 1% d'albumine sérique bovine.
6. Les cellules sont distribuées en nombre constant dans les inserts et dans les puits on ajoute du milieu avec MIP-3β. Les quantités de cellules et les volumes dépendent du type d'insert utilisé et sont indiqués dans le tableau suivant :

| Type de plaque | cellules dendritiques/insert | Volume/insert | Volume/puits |
|---|---|---|---|
| X 6 | 300 000 | 1 ml | 2 ml |
| X 12 | 150 000 | 0,4 ml | 1 ml |
| X 24 | 50 000 | 0,2 ml | 0,4 ml |

7. Après 24h, les inserts sont enlevés et les cellules qui ont traversé et sont au fond des puits sont récupérées et comptées. Les résultats sont exprimés en pourcentage par rapport au nombre de cellules déposées dans l'insert.

### RESULTATS

1. LE SURNAGEANT DE CULTURE DES TACHYZOÏTES CONTIENT UNE ACTIVITE QUI DECLENCHE LA MIGRATION DES CELLULES DENDRITIQUES.

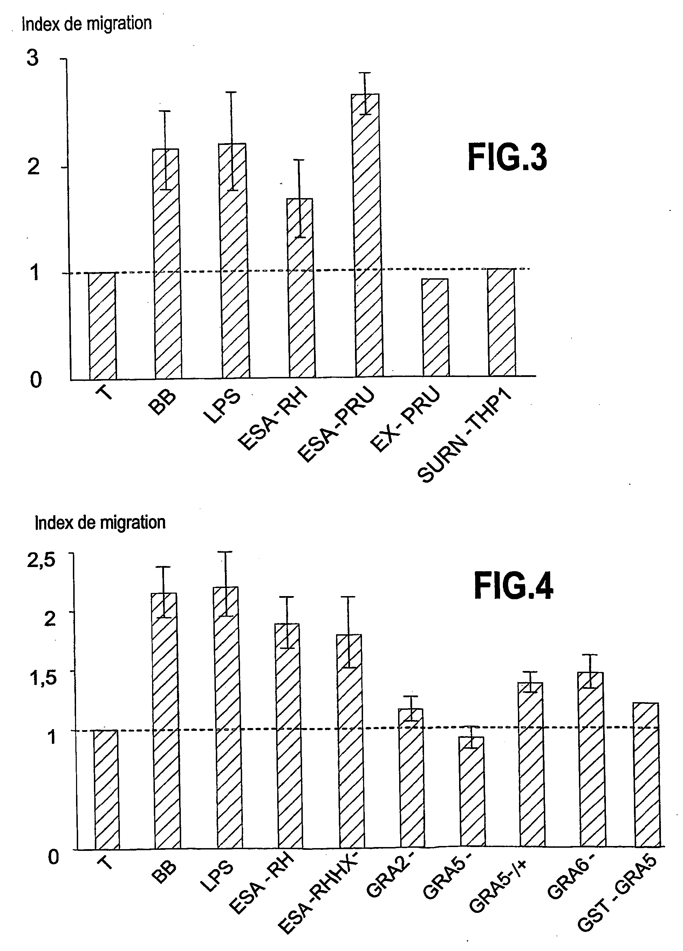

Les résultats présentés sur la **Figure 3** correspondent à un nombre d'expériences compris entre 6 et 20 selon les fractions, ils sont normalisés par le calcul d'un index de migration avec le témoin non traité (T) comme référence. Les contrôles positifs BB et LPS correspondent aux cellules dendritiques activées par la base de Bandrowski (un allergène chimique) et par le LPS (une molécule d'origine bactérienne). Les écarts types ont été calculés lorsque plus de trois expériences différentes ont été effectuées.

Le surnageant de culture des tachyzoïtes de la souche RH (ESA-RH) et celui de la souche PRU (ESA-PRU) déclenchent la migration des cellules dendritiques comme il est indiqué sur la figure.

Un extrait soluble de tachyzoïtes PRU (EX-PRU) obtenu par 3 cycles de congélation-décongélation puis élimination des molécules insolubles par centrifugation est inactif. Un surnageant de culture des cellules de la lignée humaine THP1 qui a servi à produire les tachyzoïtes *in vitro* est également inactif (SURN-THP1).

### 2. L'ACTIVITE EST CONTROLEE PAR LE GENE GRA5

La **Figure 4** montre les résultats de l'induction de la migration par les surnageants des souches RH et RH HX- qui tous deux déclenchent une migration dans 11 expériences différentes. A partir de la souche RH HX- il a été produit trois souches invalidées respectivement pour les gènes GRA2, GRA5 ou GRA6 (Mercier *et al.,* 2001, *supra).* Seuls les surnageants GRA2- et GRA6- permettent d'induire une migration significativement différente du témoin, la souche invalidée pour le gène GRAS ne permet plus de retrouver l'activité. Par conséquent, les produits de sécrétion de la souche GRA5 mutante ont perdu leur capacité d'induire la migration des cellules dendritiques.

Le gène GRAS-RH est réintroduit associé au gène de la GST dans la souche invalidée GRAS- (Mercier *et al.,* 2001, *supra)* et la nouvelle souche notée GRA5-/+ permet de produire un surnageant qui est actif. Les résultats précédents ont été reproduits dans 5 expériences différentes. Ces données permettent d'impliquer le gène GRAS dans l'activité de déclenchement de la migration.

### 3. 3.LA PARTIE N-TERMINALE DE LA MOLECULE SUPPORTE L'ACTIVITE

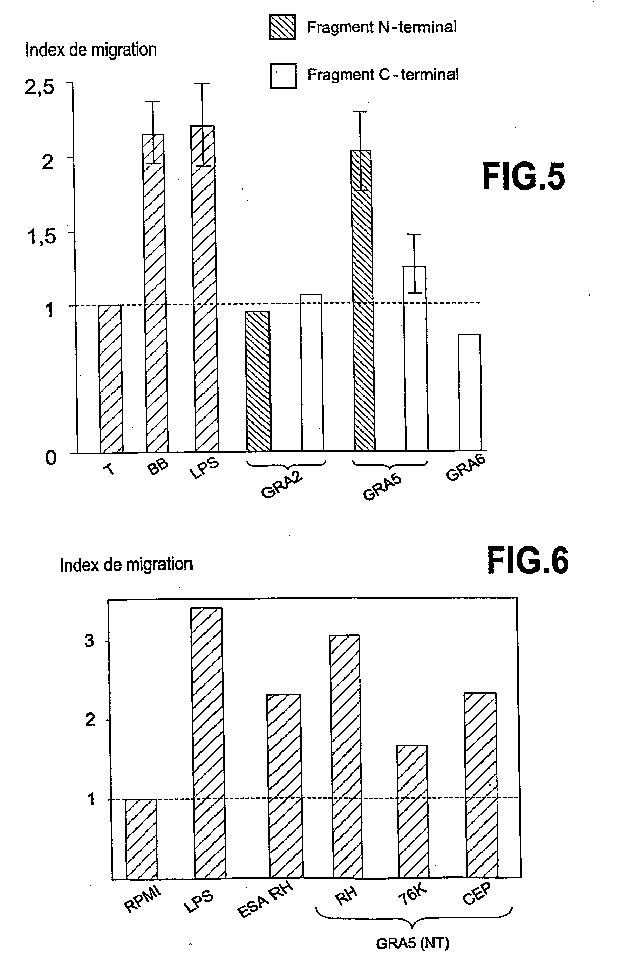

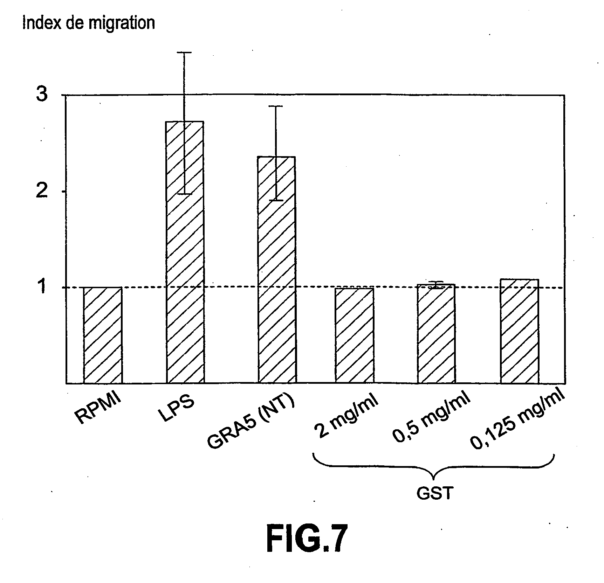

Les protéines de fusion avec la GST de la partie N-terminale de la protéine GRAS préparées précédemment (**SEQ ID N°13, 14** et **15** en ce qui concerne la partie N-terminale) ont été introduites dans le test de migration des cellules dendritiques. De façon analogue, les protéines de fusion de la GST avec la partie *C*-terminale de la protéine GRAS, ainsi que celles avec les parties *C*- et *N-* terminale des protéines GRA2 et GRA6 ont été produites et testées. La **Figure 5** donne les indexes de migration obtenus avec ces protéines de fusion (notées GRAS, GRA2 et GRA6 sur la figure) pour la souche RH. Comme il est indiqué sur la **Figure 5****,** seule la partie *N*-terminale de GRAS possède l'activité. La Figure 6 illustre les indexes de migration obtenus avec les protéines de fusion de **SEQ ID N°13, 14** et **15** correspondant respectivement aux souches RH, 76K et CEP. De plus, il a été démontrée que l'activité n'est pas supportée par la séquence GST ajoutée aux différents fragments pour faciliter leur production en système bactérien et leur purification pour deux raisons : la molécule GST par elle-même n'a pas d'activité comme le montre la **Figure 7**, enfin le fragment C-terminal de GRAS est sans activité de même que les fragments de GRA2 et GRA6 (Figure 5). Un autre élément indiquant que l'activité est supporté par la partie *N*-Terminale de GRA5 est le fait que les fragments de diverses souches ont des activités variables comme il est indiqué sur la **Figure 6****.**

### SEQUENCE DES PROTEINES D'INTERET

### SEQUENCE DE LA PROTEINE GRA5 ENTIERE A PARTIR DE LA SOUCHE RH

SEQ ID N°1
MASVKRVVVA VMIVNVLALI FVGVAGSTRD VGSGGDDSEG ARGREQQQVQ QHEQNEDRSL
FERGRAAVTG HPVRTAVGLA AAVVAVVSLL RLLKRRRRRA IQEESKESAT AEEEEVAEEE

### SEQUENCE DU FRAGMENT ACTIF N-TERMINAL

### SOUCHE RH

**SEQ ID N°3**
   GSTRDVGSGG DDSEGA**R**G**R**E QQQVQQH**E**Q**N** EDRSLFERGR AAVTGHPVRT

### SOUCHE PRU

**SEQ ID N°4**
   GSTRD**T**GSGG DDSEGA**W**GGE QQQVQQH**G**Q**S** EDRSLFERGR AAVTGHPVRT

### SOUCHE 76K

**SEQ ID N°5**
   GST**C**D**T**GSGG DDSEGAWG**G**E QQQVQQH**G**Q**S** EDRSLFERGR AAVTGHPVRT

### SOUCH_{E} C56 ET CEP

**SEQ ID N° 6**
   GSTRDVGSG**A** DDSEGA**G**GRE **R**QQVQQHEQN EDRSLFERGR AAVTGHPVRT

Différents tests ont été réalisés avec chacun de ces polypeptides et montrent que ces polypeptides provoquent la migration des cellules dendritiques et des cellules de Langerhans. On observe par immunomarquages des cellules que l'addition de chacun de ces polypeptides :
1. provoque la baisse de l'E-cadhérine sur la membrane (molécule impliquée dans les interactions inter-cellulaires dans l'épiderme).
2. provoque la perte du CCR6, récepteur de la chimiokine MIP-3α, produite dans l'épiderme et qui stabilise les cellules dendritiques à cet endroit.
3. provoque l'apparition du CCR7, récepteur de la chimiokine MIP-3β, produite dans les ganglions et qui attire les cellules dendritiques hors de l'épiderme.
4. doit provoquer la synthèse de métalloprotéases, ce dernier résultat est déduit du fait que les cellules dendritiques migrent même lorsque les inserts sont traités par le Matrigel. Le Matrigel à la composition d'une membrane basale dermo-épidermique et *in vivo,* les cellules dendritiques doivent synthétiser des métalloprotéases pour la traverser (Vincent et al. Eur. J. Cell. Biol. 2002, 81, 383-389).

L'ensemble de ces éléments met en évidence que les cellules dendritiques ont la capacité de se désolidariser de leur environnement et de traverser la membrane basale pour aller vers les ganglions drainants.

### ACTIVITE DES FRAGMENTS DE GRAS-PRU

### Les polypeptides de séquences :

**SEQ ID N°16** : DTGSGGDDSEGAWGGEQQQVQQHGQSEDR
**SEQ ID N°17** : DSEGAWGGEQQQVQQHGQSEDRSLFER
ont été préparés par synthèse peptidique.

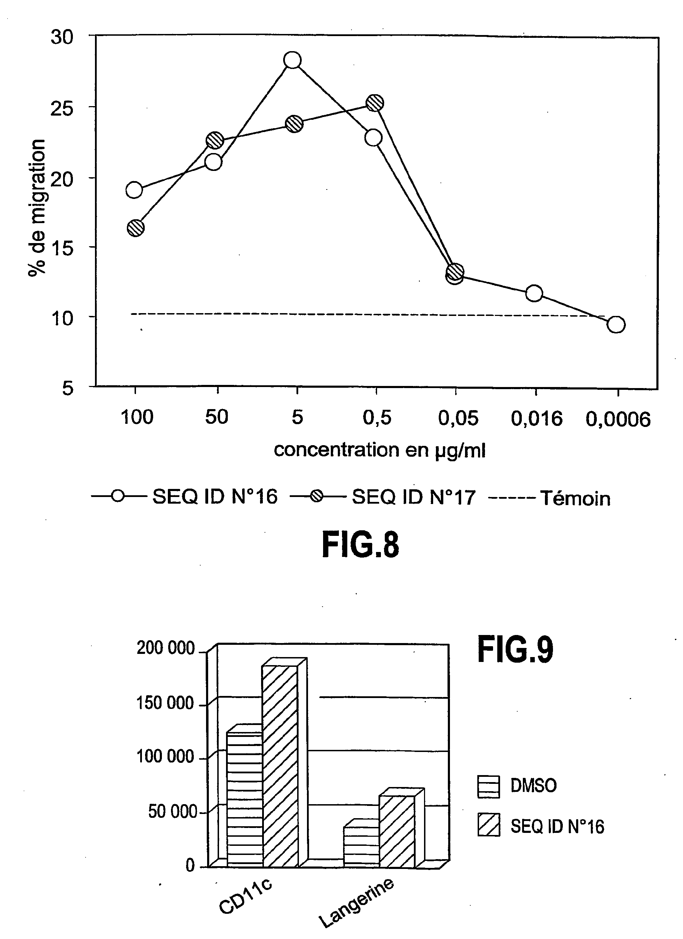

Ces peptides ont ensuite été soumis au test sur la migration des cellules dendritiques précédemment décrit. Les cellules dendritiques générées *in vitro* à partir de précurseurs isolés du sang de cordon ont été incubées la nuit en présence de doses décroissantes du peptide et le lendemain leur capacité à migrer vers la chimiokine MIP-3(3 a été testée. La **figures 8** présente les activités de déclenchement de migration *in vitro* du peptide de **SEQ ID N°16** et du peptide de **SEQ ID N°17** (un point représente la moyenne de la migration observée pour 5 expériences) en fonction de leur concentration.

Le peptide de **SEQ ID N°16** a également été testé *in vivo* chez la souris, sur un test de déclenchement de la migration. Pour cela, a été utilisé un lot de souris chez lesquelles le gène EGFP est introduit devant le gène de la Langerine, ce qui rend les cellules de Langerhans naturellement fluorescentes. Ces souris également sont invalidées pour le gène codant pour le CCR6 mais les cellules de Langerhans sont présentes dans l'épiderme en nombre habituel. Ces souris sont obtenues après croisement entre deux souches de souris : la lignée Langerine DTR EGFP (Kissenpfennig A, et al. dans Trends Immunol. 2006 Mar; 27(3):132-9. Immunity. 2005 May; 22(5):643-54) maintenue au Centre de distribution, typage et archivage du CNRS à Orléans, France et disponible après de l'European Mouse Mutant Archive (EMMA, GSF National Research Center for Environment and Health, Institute of Experimental Genetics, Ingolstädter Landstrasse 1, D-85764 Neuherberg, Munich, Allemagne,www.emmanet.org) sous le numéro identifiant EM:01822 et la lignée invalidée pour le gène CCR6, publiée par Cook et al. dans Immunity, 2000, 12 : 495-503 et par Lukrs et al. dans J. Exp. Med., 2001,194, 551-555.

Le peptide de **SEQ ID N°16** est préparé dans le DMSO à 25mg/ml. On applique 5 µl de cette préparation sur l'oreille gauche de la souris et un volume équivalent seul sur l'oreille droite. 24h plus tard, la souris est sacrifiée et les ganglions auriculaires sont prélevés, le nombre de cellules de Langerhans (naturellement fluorescentes) est compté et le nombre total de cellules dendritiques repéré par un marquage avec un anticorps anti-CD11c.

Un plus grand nombre de cellules dendritiques et de cellules de Langerhans est observé, dans les ganglions gauches que dans les droits, ce qui indique que le peptide de **SEQ ID N°16** déclenche la migration de ces cellules *in vivo.* La **figure 9** montre le nombre de cellules de Langerhans et de cellules dendritiques repéré par un marquage avec un anticorps anti-CD11c, obtenu du côté du traitement avec le peptide de **SEQ** I**D N°16** et du côté du traitement au DMSO seul.

## Revendications

1. Polypeptide correspondant aux acides aminés 26 à 75 de la séquence de la protéine GRA5-RH de **SEQ ID N°1** : 
MASVKRVVVAVMIVNVLALIFVGVAGSTRDVGSGGDDSEGARGRE
QQQVQQHEQNEDRSLFERGRAAVTGHPVRTAVGLAAAVVAVVSLL
RLLKRRRRRAIQEESKESATAEEEEVAEEE
 ses variants ou homologues induisant la migration des cellules dendritiques et qui présentent au moins 95 % d'homologie avec les séquences d'acides aminés des polypeptides dont ils sont le variant ou l'homologue, ainsi que leurs fragments induisant la migration des cellules dendritiques et qui comportent au minimum 15 acides aminés consécutifs.

2. Polypeptides selon la revendication 1 choisis parmi les polypeptides de séquence :
**SEQ ID N°3**
GSTRDVGSGG DDSEGARGRE QQQVQQHEQN EDRSLFERGR AAVTGHPVRT
**SEQ ID N°4**
GSTRDTGSGG DDSEGAWGGE QQQVQQHGQS EDRSLFERGR AAVTGHPVRT
**SEQ ID N°5**
GSTCDTGSGG DDSEGAWGGE QQQVQQHGQS EDRSLFERGR AAVTGHPVRT
**SEQ ID N°6**
GSTRDVGSGA DDSEGAGGRE RQQVQQHEQN EDRSLFERGR AAVTGHPVRT
leurs homologues qui présentent au moins 95 % d'homologie avec les séquences d'acides aminés des polypeptides dont ils sont l'homologue ou fragments qui comportent au minimum 15 acides aminés consécutifs, induisant la migration des cellules dendritiques.

3. Polypeptides selon la revendication 1 ou 2 choisis parmi les polypeptides de séquence :
**SEQ ID N°3**
GSTRDVGSGG DDSEGARGRE QQQVQQHEQN EDRSLFERGR AAVTGHPVRT
**SEQ ID N°4**
GSTRDTGSGG DDSEGAWGGE QQQVQQHGQS EDRSLFERGR AAVTGHPVRT
**SEQ ID N°5**
GSTCDTGSGG DDSEGAWGGE QQQVQQHGQS EDRSLFERGR AAVTGHPVRT
**SEQ ID N°6**
GSTRDVGSGA DDSEGAGGRE RQQVQQHEQN EDRSLFERGR AAVTGHPVRT

4. Polypeptides selon la revendication 1 ou 2 choisis parmi les fragments correspondant aux acides aminés 30 à 58, ou 37 à 63 de la séquence d'une protéine GRAS, et en particulier de la séquence d'une protéine GRAS des souches RH, PRU, 76K, CR6 ou CEP, leurs homologues induisant la migration des cellules dendritiques et qui présentent au moins 95 % d'homologie avec les séquences d'acides aminés des polypeptides dont ils sont l'homologue, ainsi que leurs fragments induisant la migration des cellules dendritiques et qui comportent au minimum 15 acides aminés consécutifs.

5. Polypeptides selon la revendication 4 choisis parmi les polypeptides de séquence :
**SEQ ID N°16** : DTGSGGDDSEGAWGGEQQQVQQHGQSEDR
**SEQ ID N°17** : DSEGAWGGEQQQVQQHGQSEDRSLFER
leurs homologues induisant la migration des cellules dendritiques et qui présentent au moins 95 % d'homologie avec les séquences d'acides aminés des polypeptides dont ils sont l'homologue, ainsi que leurs fragments induisant la migration des cellules dendritiques et qui comportent au minimum 15 acides aminés consécutifs.

6. Acide nucléique purifié ou isolé constitué par un enchaînement de nucléotides codant pour un polypeptide selon l'une des revendications 1 à 5.

7. Protéine de fusion entre la GST et un polypeptide selon l'une des revendications 1 à 5 ainsi que les homologues qui présentent au moins 95 % d'homologie avec les séquences d'acides aminés des polypeptides dont ils sont l'homologue ou fragments de telles protéines qui comportent au minimum 15 acides aminés consécutifs, induisant la migration des cellules dendritiques.

8. Protéine selon la revendication 7 choisies parmi la protéine de **SEQ ID N°13 :** 
S P I L G Y W K I K G L V Q P T R L L L E Y L E E K Y E E H L Y E R D E G D K W R N K
K F E L G L E F P N L P Y Y I D G D V K L T Q S M A I I R Y I A D K H N M L G G C P K
E R A E I S M L E G A V L D I R Y G V S R I A Y S K D F E T L K V D F L S K L P E M L K
M F E D R L C H K T Y L N G D H V T H P D F M L Y D A L D V V L Y M D P M C L D A F
P K L V C F K K R I E A I P Q I D K Y L K S S K Y I A W P L Q G W Q A T F G G G D H P
P K S D L I E G R G I P G G S T R D V G S G G D D S E G A R G R E Q Q V Q Q H E Q
N E D R S L F E R G R A A V T G H P V R E F I V T D
 la protéine de **SEQ ID N°14 :** 
S P I L G Y W K I K G L V Q P T R L L L E Y L E E K Y E E H L Y E R D E G D K W R N K
K F E L G L E F P N L P Y Y I D G D V K L T Q S M A I I R Y I A D K H N M L G G C P K
E R A E I S M L E G A V L D I R Y G V S R I A Y S K D F E T L K V D F L S K L P E M L K
M F E D R L C H K T Y L N G D H V T H P D F M L Y D A L D V V L Y M D P M C L D A F
P K L V C F K K R I E A I P Q I D K Y L K S S K Y I A W P L Q G W Q A T F G G G D H P
P K S D L I E G R G I P G G S T C D T G S G G D D S E G A W G G E Q Q V Q Q H G Q
S E D R S L F E R G R A A V T G H P V R E F I V T D
 la protéine de **SEQ ID N°15 :** 
S P I L G Y W K I K G L V Q P T R L L L E Y L E E K Y E E H L Y E R D E G D K W R N K
K F E L G L E F P N L P Y Y I D G D V K L T Q S M A I I R Y I A D K H N M L G G C P K
E R A E I S M L E G A V L D I R Y G V S R I A Y S K D F E T L K V D F L S K L P E M L K
M F E D R L C H K T Y L N G D H V T H P D F M L Y D A L D V V L Y M D P M C L D A F
P K L V C F K K R I E A I P Q I D K Y L K S S K Y I A W P L Q G W Q A T F G G G D H P
P K S D L I E G R G I P G G S T R D V G S G A D D S E G A G G R E R Q Q V Q Q H E Q
N E D R S L F E R G R A A V T G H P V R E F I V T D
 ainsi que les homologues qui présentent au moins 95 % d'homologie avec les séquences d'acides aminés des polypeptides dont ils sont l'homologue ou fragments de telles protéines qui comportent au minimum 15 acides aminés consécutifs, induisant la migration des cellules dendritiques.

9. Polypeptides selon l'une des revendications 1 à 5, en tant que médicaments.

10. Compositions pharmaceutiques, en particulier adaptées à une administration topique, comprenant un polypeptide selon l'une des revendications 1 à 5, ou une protéine de fusion selon la revendication 7 ou 8, en combinaison avec au moins un excipient pharmaceutiquement acceptable.

11. Utilisation d'un polypeptide selon l'une des revendications 1 à 5, ou une protéine de fusion selon la revendication 7 ou 8 pour la fabrication d'un médicament destiné à prévenir ou traiter des pathologies de la peau ou des muqueuses impliquant des cellules dendritiques ou des cellules de Langerhans.

12. Utilisation d'un polypeptide selon l'une des revendications 1 à 5, ou une protéine de fusion selon la revendication 7 ou 8, pour la fabrication d'un médicament destiné à prévenir ou traiter des pathologies choisies parmi : la dermatite atopique, les inflammations, les maladies auto-immunes, le Lupus érythémateux disséminé, la polyarthrite, le psoriasis, la réaction du greffon contre l'hôte, le rejet de greffe, ou les allergies, telles que les allergies de contact, par exemple au latex, aux cosmétiques, aux parfums, aux tatouages, aux métaux et à leurs sels, les phénomènes allergiques pulmonaires dues aux allergènes aériens, l'asthme, les rhinites allergiques ou les allergies alimentaires.

## Claims

1. Polypeptide corresponding to amino acids 26 to 75 of the sequence of the GRA5-RH protein of SEQ ID NO.1: 
MASVKRVVVAVMIVNVLALIFVGVAGSTRDVGSGGDDSEGARGRE
QQQVQQHEQNEDRSLFERGRAAVTGHPVRTAVGLAAAVVAVVSLL
RLLKRRRRRAIQEESKESATAEEEEVAEEE
 variants or homologues thereof that induce the migration of dendritic cells and which have at least 95% homology with the amino acid sequences of the polypeptides of which they are variants or homologues, and fragments thereof that induce the migration of dendritic cells and consist of a minimum of 15 consecutive amino acids.

2. Polypeptides according to claim 1 selected from the polypeptides of sequence:
SEQ ID NO.3
GSTRDVGSGG DDSEGARGRE QQQVQQHEQN EDRSLFERGR AAVTGHPVRT
SEQ ID NO.4
GSTRDTGSGG DDSEGAWGGE QQQVQQHGQS EDRSLFERGR AAVTGHPVRT
SEQ ID NO.5
GSTCDTGSGG DDSEGAWGGE QQQVQQHGQS EDRSLFERGR AAVTGHPVRT
SEQ ID NO.6
GSTRDVGSGA DDSEGAGGRE RQQVQQHEQN EDRSLFERGR AAVTGHPVRT
homologues thereof which have at least 95% homology with the amino acid sequences of the polypeptides of which they are homologues or fragments thereof consisting of a minimum of 15 consecutive amino acids, that induce the migration of dendritic cells.

3. Polypeptides according to claim 1 or 2 selected from the polypeptides of sequence:
SEQ ID NO.3
GSTRDVGSGG DDSEGARGRE QQQVQQHEQN EDRSLFERGR AAVTGHPVRT
SEQ ID NO.4
GSTRDTGSGG DDSEGAWGGE QQQVQQHGQS EDRSLFERGR AAVTGHPVRT
SEQ ID NO.5
GSTCDTGSGG DDSEGAWGGE QQQVQQHGQS EDRSLFERGR AAVTGHPVRT
SEQ ID NO.6
GSTRDVGSGA DDSEGAGGRE RQQVQQHEQN EDRSLFERGR AAVTGHPVRT

4. Polypeptides according to claim 1 or 2 selected from the fragments corresponding to amino acids 30 to 58, or 37 to 63 of the sequence of a GRAS protein, and in particular of the sequence of a GRA5 protein from the strains RH, PRU, 76K, CR6 or CEP, homologues thereof that induce the migration of dendritic cells and which have at least 95% homology with the amino acid sequences of the polypeptides of which they are homologues, as well as fragments thereof that induce the migration of dendritic cells and consist of a minimum of 15 consecutive amino acids.

5. Polypeptides according to claim 4 selected from the polypeptides of sequence:
SEQ ID NO.16: DTGSGGDDSEGAWGGEQQQVQQHGQSEDR
SEQ ID NO.17: DSEGAWGGEQQQVQQHGQSEDRSLFER
homologues thereof that induce the migration of dendritic cells and which have at least 95% homology with the amino acid sequences of the polypeptides of which they are homologues, as well as fragments thereof that induce the migration of dendritic cells and consist of a minimum of 15 consecutive amino acids.

6. Purified or isolated nucleic acid consisting of a sequence of nucleotides encoding a polypeptide according to claims 1 through 5.

7. Fusion protein between GST and a polypeptide according to one of claims 1 through 5 as well as the homologues which have at least 95% homology with the amino acid sequences of the polypeptides of which they are homologues or fragments of such proteins consisting of a minimum of 15 consecutive amino acids, that induce the migration of dendritic cells.

8. Protein according to claim 7 selected from:
the protein of SEQ ID NO.13: 
S P I L G Y W K I K G L V Q P T R L L L E Y L E E K Y E E H L
Y E R D E G D K W R N K K F E L G L E F P N L P Y Y I D G D V
K L T Q S M A I I R Y I A D K H N M L G G C P K E R A E I S M
L E G A V L D I R Y G V S R I A Y S K D F E T L K V D F L S K
L P E M L K M F E D R L C H K T Y L N G D H V T H P D F M L Y
D A L D V V L Y M D P M C L D A F P K L V C F K K R I E A I P
Q I D K Y L K S S K Y I A W P L Q G W Q A T F G G G D H P P K
S D L I E G RG I P G G S T R D V G S G G D D S E G A R G R E Q
Q Q V Q Q H E Q N E D R S L F E R G R A A V T G H P V R E F I
V T D
the protein of SEQ ID NO.14: 
S P I L G Y W K I K G L V Q P T R L L L E Y L E E K Y E E H L
Y E R D E G D K W R N K K F E L G L E F P N L P Y Y I D G D V
K L T Q S M A I I R Y I A D K H N M L G G C P K E R A E I S M
L E G A V L D I R Y G V S R I A Y S K D F E T L K V D F L S K
L P E M L K M F E D R L C H K T Y L N G D H V T H P D F M L Y
D A L D V V L Y M D P M C L D A F P K L V C F K K R I E A I P
 
Q I D K Y L K S S K Y I A W P L Q G W Q A T F G G G D H P P K
S D L I E G RG I P G G S T C D T G S G G D D S E G A W G G E Q
Q Q V Q Q H G Q S E D R S L F E R G R A A V T G H P V R E F I
V T D
the protein of SEQ ID NO.15: 
S P I L G Y W K I K G L V Q P T R L L L E Y L E E K Y E E H L
Y E R D E G D K W R N K K F E L G L E F P N L P Y Y I D G D V
K L T Q S M A I I R Y I A D K H N M L G G C P K E R A E I S M
L E G A V L D I R Y G V S R I A Y S K D F E T L K V D F L S K
L P E M L K M F E D R L C H K T Y L N G D H V T H P D F M L Y
D A L D V V L Y M D P M C L D A F P K L V C F K K R I E A I P
Q I D K Y L K S S K Y I A W P L Q G W Q A T F G G G D H P P K
S D L I E G RG I P G G S T R D V G S G A D D S E G A G G R E R
Q Q V Q Q H E Q N E D R S L F E R G R A A V T G H P V R E F I
V T D
as well as the homologues which have at least 95% homology with the amino acid sequences of the polypeptides of which they are homologues or fragments of such proteins consisting of a minimum of 15 consecutive amino acids, that induce the migration of dendritic cells.

9. Polypeptides according to one of claims 1, through 5, as medicinal products.

10. Pharmaceutical compositions, particularly those suitable for topical administration, containing a polypeptide according to one of claims 1 through 5, or a fusion protein according to claim 7 or 8, in combination with at least one pharmaceutically suitable excipient.

11. The use of a polypeptide according to one of claims 1 through 5, or a fusion protein according to claim 7 or 8, for the manufacture of a medicinal product intended for the prevention or treatment of diseases of the skin or mucosae in which dendritic cells or Langerhans cells are implicated.

12. The use of a polypeptide according to one of claims 1 through 5, or a fusion protein according to claim 7 or 8, for the manufacture of a medicinal product intended for the prevention or treatment of diseases selected from: atopic dermatitis, inflammations, autoimmune diseases, systemic lupus erythematosus, polyarthritis, psoriasis, graft versus host disease, graft rejection, or allergies, such as contact allergies, for example to latex, cosmetics, perfumes, tattoos, metals and their salts, pulmonary allergic phenomena due to airborne allergens, asthma, allergic rhinitis or food allergies.

## Patentansprüche

1. Polypeptid, das den Aminosäuren 26 bis 75 der Sequenz des GRA5-RH Proteins mit der SEQ ID Nr. 1 entspricht: 
MASVKRVVVAVMIVNVLALIFVGVAGSTRDVGSGGDDSEGARGRE
QQQVQQHEQNEDRSLFERGRAAVTGHPVRTAVGLAAAVVAVVSLL
RLLKRRRRRAIQEESKESATAEEEEVAEEE
 seine Varianten oder Homologe, die die Migration der dendritischen Zellen induzieren und die eine Homologie von wenigstens 95 % mit den Aminosäuresequenzen der Polypeptide, deren Variante oder Homolog sie sind, aufweisen, sowie ihre Fragmente, die die Migration der dendritischen Zellen induzieren und die mindestens 15 aufeinanderfolgende Aminosäuren umfassen.

2. Polypeptide nach Anspruch 1, die ausgewählt sind aus den Polypeptiden mit der Sequenz:
SEQ ID Nr. 3
GSTRDVGSGG DDSEGARGRE QQQVQQHEQN EDRSLFERGR AAVTGHPVRT
SEQ ID Nr. 4
GSTRDTGSGG DDSEGAWGGE QQQVQQHGQS EDRSLFERGR AAVTGHPVRT
SEQ ID Nr. 5
GSTCDTGSGG DDSEGAWGGE QQQVQQHGQS EDRSLFERGR AAVTGHPVRT
SEQ ID Nr. 6
GSTRDVGSGA DDSEGAGGRE RQQVQQHEQN EDRSLFERGR AAVTGHPVRT
ihren Homologen, die eine Homologie von wenigstens 95 % mit den Aminosäuresequenzen der Polypeptide, deren Homolog sie sind, aufweisen, oder Fragmenten mit mindestens 15 aufeinanderfolgenden Aminosäuren, die die Migration der dendritischen Zellen induzieren.

3. Polypeptide nach Anspruch 1 oder 2, die ausgewählt sind aus den Polypeptiden mit der Sequenz:
SEQ ID Nr. 3
GSTRDVGSGG DDSEGARGRE QQQVQQHEQN EDRSLFERGR AAVTGHPVRT
SEQ ID Nr. 4
GSTRDTGSGG DDSEGAWGGE QQQVQQHGQS EDRSLFERGR AAVTGHPVRT
SEQ ID Nr. 5
GSTCDTGSGG DDSEGAWGGE QQQVQQHGQS EDRSLFERGR AAVTGHPVRT
SEQ ID Nr. 6
GSTRDVGSGA DDSEGAGGRE RQQVQQHEQN EDRSLFERGR AAVTGHPVRT

4. Polypeptide nach Anspruch 1 oder 2, die ausgewählt sind aus den Fragmenten, die den Aminosäuren 30 bis 58 oder 37 bis 63 der Sequenz eines GRA5-Proteins und insbesondere der Sequenz eines GRA5-Proteins der Typen RH, PRU, 76K, CR6 oder CEP entsprechen, ihren Homologen, die die Migration der dendritischen Zellen induzieren und die eine Homologie von wenigstens 95 % mit den Aminosäuresequenzen der Polypeptide, deren Homolog sie sind, aufweisen, sowie ihren Fragmenten, die die Migration der dendritischen Zellen induzieren und die mindestens 15 aufeinanderfolgende Aminosäuren umfassen.

5. Polypeptide nach Anspruch 4, die ausgewählt sind aus den Polypeptiden mit der Sequenz:
SEQ ID Nr. 16: DTGSGGDDSEGAWGGEQQQVQQHGQSEDR
SEQ ID Nr 17: DSEGAWGGEQQQVQQHGQSEDRSLFER
ihren Homologen, die die Migration der dendritischen Zellen induzieren und die eine Homologie von wenigstens 95 % mit den Aminosäuresequenzen der Polypeptide, deren Homolog sie sind, aufweisen, sowie ihren Fragmenten, die die Migration der dendritischen Zellen induzieren und die mindestens 15 aufeinanderfolgende Aminosäuren umfassen.

6. Gereinigte oder isolierte Nukleinsäure, die aus einer Aneinanderreihung von Nukleotiden, welche für ein Polypeptid nach einem der Ansprüche 1 bis 5 codieren, besteht.

7. Fusionsprotein zwischen GST und einem Polypeptid nach einem der Ansprüche 1 bis 5 sowie die Homologe, die eine Homologie von wenigstens 95 % mit den Aminosäuresequenzen der Polypeptide, deren Homolog sie sind, aufweisen, oder Fragmente derartiger Proteine, mit mindestens 15 aufeinanderfolgenden Aminosäuren, die die Migration der dendritischen Zellen induzieren.

8. Protein nach Anspruch 7, das ausgewählt ist aus
dem Protein mit der SEQ ID Nr. 13: 
SPILGYWKIKGLVQPTRLLLEYLEEKYEEHLYERDEGDKWRNK
KFELGLEFPNLPYYIDGDVKLTQSMAIIRYIADKHNMLGGCPKE
RAEISMLEGAVLDIRYGVSRIAYSKDFETLKVDFLSKLPEMLKM
FEDRLCHKTYLNGDHVTHPDFMLYDALDVVLYMDPMCLDAFPK
LVCFKKRIEAIPQIDKYLKSSKYIAWPLQGWQATFGGGDHP
PKSDLIEGRGIPGGSTRDVGSGGDDSEGARGREQQVQQHEQ
NEDRSLFERGRAAVTGHPVREFIVTD
 dem Protein mit der SEQ ID Nr. 14: 
SPILGYWKIKGLVQPTRLLLEYLEEKYEEHLYERDEGDKWRNK
KFELGLEFPNLPYYIDGDVKLTQSMAIIRYIADKHNMLGGCPKE
RAEISMLEGAVLDIRYGVSRIAYSKDFETLKVDFLSKLPEMLKM
FEDRLCHKTYLNGDHVTHPDFMLYDALDVVLYMDPMCLDAFPK
LVCFKKRIEAIPQIDKYLKSSKYIAWPLQGWQATFGGGDHP
PKSDLIEGRGIPGGSTCDTGSGGDDSEGAWGGEQQVQQHG
QSEDRSLFERGRAAVTGHPVREFIVTD
 dem Protein mit der SEQ ID Nr. 15: 
SPILGYWKIKGLVQPTRLLLEYLEEKYEEHLYERDEGDKWRNK
KFELGLEFPNLPYYIDGDVKLTQSMAIIRYIADKHNMLGGCPKE
RAEISMLEGAVLDIRYGVSRIAYSKDFETLKVDFLSKLPEMLKM
FEDRLCHKTYLNGDHVTHPDFMLYDALDVVLYMDPMCLDAFPK
LVCFKKRIEAIPQIDKYLKSSKYIAWPLQGWQATFGGGDHP
PKSDLIEGRGIPGGSTRDVGSGADDSEGAGGRERQQVQQHEQ
NEDRSLFERGRAAVTGHPVREFIVTD
 sowie den Homologen, die eine Homologie von wenigstens 95 % mit den Aminosäuresequenzen der Polypeptide, deren Homolog sie sind, aufweisen, oder Fragmenten derartiger Proteine, mit mindestens 15 aufeinanderfolgenden Aminosäuren, die die Migration der dendritischen Zellen induzieren.

9. Polypeptide nach einem der Ansprüche 1 bis 5, als Medikamente.

10. Pharmazeutische Zusammensetzungen, die insbesondere für eine topische Verabreichung geeignet sind, umfassend ein Polypeptid nach einem der Ansprüche 1 bis 5 oder ein Fusionsprotein nach Anspruch 7 oder 8, in Kombination mit wenigstens einem pharmazeutisch verträglichen Hilfsstoff.

11. Verwendung eines Polypeptids nach einem der Ansprüche 1 bis 5 oder eines Fusionsproteins nach Anspruch 7 oder 8 für die Herstellung eines Medikaments, das dazu bestimmt ist, Phatologien der Haut oder der Schleimhäute, an denen dendritische Zellen oder Langerhans-Zellen beteiligt sind, vorzubeugen oder zu behandeln.

12. Verwendung eines Polypeptids nach einem der Ansprüche 1 bis 5 oder eines Fusionsproteins nach Anspruch 7 oder 8, für die Herstellung eines Medikaments, das dazu bestimmt ist, Pathologien vorzubeugen oder zu behandeln, die ausgewählt sind aus atopischer Dermatitis, Entzündungen, Autoimmunerkrankungen, disseminiertem Lupus erythematodes, Polyarthritis, Psoriasis, Transplantat-gegen-Wirt-Reaktion, Transplantatabstoßung oder aus Allergien, wie Kontaktallergien, beispielsweise auf Latex, Kosmetika, Parfum, Tätowierungen, Metalle und ihre Salze, allergischen Lungenerscheinungen, die durch Luftallergene bedingt sind, Asthma, allergische Schnupfen oder Nahrungsmittelallergien.
